# EUROPEAN PATENT APPLICATION

(11) **EP 2 561 885 A2**
(43) Date of publication of application: **27.02.2013**
(21) Application number: 11772243.9
(22) Date of filing: 21.04.2011
(51) Int. Cl.: A61K 38/16, A61K 38/10, A61K 39/395, A61P 35/00

(54) **USE OF HADES AS TUMOR SUPPRESSOR TARGET**

(30) Priority: 21.04.2010 US 326245 P
(71) Applicant: Konkuk University Industrial Cooperation Corp., Seoul 143-701 (KR)
(72) Inventor: AN, Sungkwan, Seoul 143-190 (KR); JUNG, Jin Hyuk, Seoul 138-790 (KR); LEE, Jae Ho, Uijeongbu-si Gyeonggi-do 480-944 (KR); BAE, Seunghee, Seoul 130-070 (KR)
(74) Representative: Winkler, Andreas Fritz Ernst
(86) International application number: PCT/KR2011/002872
(87) International publication number: WO 2011/132955

(57) **Abstract**

The present invention relates to a new use of Hades as a tumor suppressor target, more particularly to a composition for suppressing tumor comprising an expression or action inhibitor of Hades protein having an amino acid sequence of SEQ ID NO: 2 as an effective ingredient.

The present inventors have found that the overexpressed Hades protein interacts with p53 to inhibit the exonuclear mechanism of p53 and the knowdown of Hades induces increase in the expression of p53, demonstrating that Hades is a negative regulator to p53. Therefore, it would be understood that the inhibition of Hades overexpressed in tumor cells contributes to tumor-supressive effects of p53. The drug candidates capable of modulating the expression of the Hades protein, inhibiting the actions of the Hades protein or inhibiting interecation between Hades and p53 are considered a promising anticancer drug.

## Description

### [Technical Field]

The present invention relates to a new use of Hades as a tumor suppressor target, more particularly to a composition for suppressing tumor comprising an expression or action inhibitor of Hades protein having an amino acid sequence of SEQ ID NO: 2 as an effective ingredient.

### [Background Art]

Tumor suppressor p53 acts as a central switch to induce apoptosis and cell cycle arrest in response to a variety of cellular signals, including DNA damage and hypoxia. Although the functions of p53 in the nucleus have been well known to play a crucial role in cellular homeostasis and organismal survival [Dulic et al., Cell 76, 1013-1023 (1994); Lowe et al., Nature 362, 847-849 (1993); Lane DP Cancer. Nature 358, 10-10 (1992); Raycroft et al., Science 249, 1049-1051 (1990)], transcription-independent mechanisms, known as exonuclear functions, are also important for maintaining the tumor suppressor function of p53 [Green et al., Nature 458, 1127-1130 (2009)]. Several lines of evidence support an exonuclear role of p53, including p53-dependent cell death in the absence of protein synthesis and gene transcription [Wagner et al., Genes Dev. 8, 2817-2830 (1994)] and potent induction of apoptosis by a transcriptionally defective p53 mutant [Caelles et al., Nature 370, 220-223 (1994); Haupt et al., Genes Dev. 9, 2170-2183 (1995)]. In addition, activation of the cytoplasmic p53 in cell-free system induces release of mitochondrial cytochrome C [Schuler et al., J Biol Chem. 275, 7337-7342 (2000)]. p53 is translocated to the cytoplasm and mitochondria upon DNA damage [Marchenko et al., J Biol Chem. 275, 16202 (2000)] and binds BcI-xl and Bad in the cytoplasm [Mihara et al., Molecular Cell 11, 577-590 (2003)], thereby inhibiting the interaction between pro-apoptotic Bcl-2 proteins and anti-apoptotic Bcl-2 proteins and promoting oligomerization of pro-apoptotic Bcl-2 proteins [Chipuk et al., Science 303, 1010-1014 (2004); Jiang et al., Mol Cell Biol. 26, 9071-9082 (2000); Tomita et al., J Biol Chem. 281, 8600-8606 (2006)].

The mechanisms underlying regulation of cytoplasmic p53 and its exonuclear function have been partially elucidated. The p53 ubiquitination has been suggested to regulate not only p53 localization, but also its exonuclear function [Geyer et al., Nat Cell Biol. 2, 569-573 (2000); [Marchenko et al., EMBO J. 26, 923-934 (2007)]. The ubiquitinated form of p53 cannot bind Bax, while deubiquitination of p53 by the ubiquitin-specific protease HAUSP allows p53 to bind to BH3-domain protein and increases the mitochondrial permeability and cell death [Marchenko et al., Cell Cycle 6, 1718-1723 (2007)]. Thus, p53 ubiquitination inhibits the interaction between p53 and Bcl-2 proteins. Taken together, these observations suggest that p53 ubiquitination may play a central role in the exonuclear function and nucleo-cytoplasmic shuttling of p53, as well as in proteasomal degradation. However, the mechanisms that regulate the p53 exonuclear role are not fully understood.

The present inventors have made intensive to overcome shortcomings of conventional technologies described above. The present inventors have found that Hades localized in mitochondria is bound to p53 in cytosol, overexpresed in tumor cells, and the overexpressed Hades protein interacts with the DNA binding domain of p53 to inhibit functions of p53.

### [Disclosure]

### [Technical Problem]

The object of the present invention is to provide a composition for suppressing tumor comprising an expression or action inhibitor of the Hades protein which have an effect to inhibit the interaction between Hades protein and p53 as an effective ingredient.

Another object of the present invention is to provide a method for screening anti-cancer agent which inhibits the interaction between Hades protein and p53.

Another object of the present invention is to provide an anti-cancer composition comprising the agent screened by the method for screening anti-cancer agent according to the present invention as an effective ingredient.

### [Technical Solution]

According to an embodiment of the present invention, there is provided a composition for suppressing tumor comprising an expression or action inhibitor of Hades protein having an amino acid sequence of SEQ ID NO: 2 as an effective ingredient. It is first proposed in the present invention that inhibition of expression or action of Hades, a negative regulator of p53, promotes the function of p53 and may lead to enhancement of tumor suppresion.

The Hades, a protein binding to p53, contains a transmembrane (TM) domain or signal peptide in the N terminus. a second TM domain in the middle, and a RING-finger domain (i.e., the signature E3 ligase domain) in the C terminus. The Hades is localised in mitochondria.

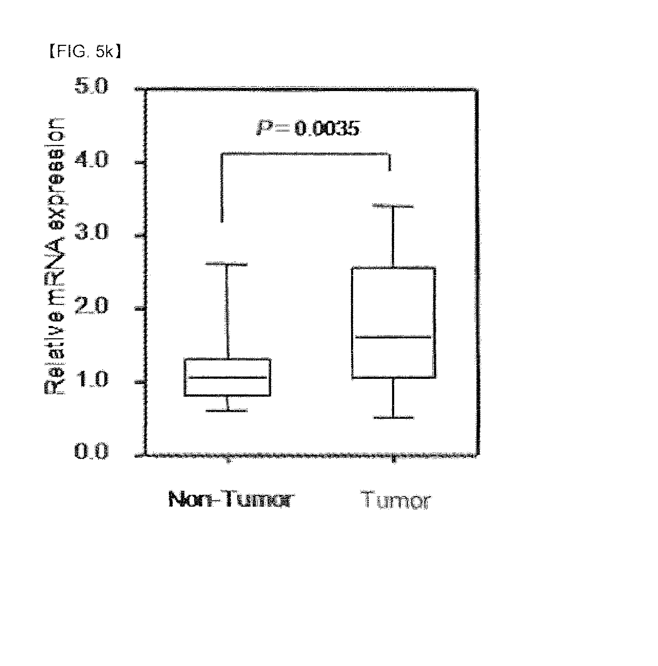

In the composition for suppressing tumor of the present invention, the Hades is over-expressed in tumor cells. In the example of the present invention, we determined the expression level of Hades in human tumor tissue and 2 fold of higher mRNA level of *Hades* was observed in human tumor tissue compared with adherent normal tissue (see Fig 5k).

In the composition for suppressing tumor of the present invention, the expression or action inhibitor of Hades protein may be any material and method which have been known to inhibit the expession or action of protein. Preferably a composition for suppressing tumor in which the expression or action inhibitor of Hades protein downregulates transcription or translation of Hades gene or inhibits action of Hades protein is provided. As used herein, "downregulation of transcription or translation of Hades gene" includes downregulation of transcription by binding to the Hades promoter gene, degradation of mRNA after transcription, interruption of translation, or any other downregulation. And, "inhibition of action of Hades protein" includes inhibition of protein activity and interruption of protein interaction with other proteins by competitively binding.

Examples of the expression inhibitor of Hades protein may includes siRNA (short interfering RNA) using RNA interference of Hades gene or shRNA (short hairpin RNA). RNA interference (hereinafter, "RNAi") is a mechanism that inhibits gene expression after transcription in many eukaryotes. RNAi is induced by short double-stranded RNA ("dsRNA") molecules existing in cells [Fire et al., Nature 391: 806-811 (1998)]. These short dsRNA molecules also known as the "siRNA" are separated into single strands and bind to "RNA-induced silencing complex (RISC)", thereby cleaving target RNA or interfering translation [Elbashir et al., Genes Dev., 15: 188-200 (2001)].

Thus, the present invention provides a separated siRNA comprising short double-stranded RNA consisting of from about 17 to about 25 nucleotides targeting mRNA of Hades gene. The siRNA comprises a sense RNA strand and its complementary antisense RNA strand. These two strands bind (anneal) with each other through Watson-Crick base pairing interaction. The sense strand includes the same nucleotide sequence in the target sequence of the target mRNA. The target sequence of siRNA may be selected by a method published in the literature, e.g., [Tuschl et al., "The siRNA User Guide" revised Oct. 11 (2002)].

The Hades target sequences used to manufacture the siRNA of the present invention are two parts, 5'-GGGAUUUUUAUCUCGAGGC-3' and 5'-CGUGUGUGUAGAGGACAAA-3', among the non-translated region in the 3' vicinity of Hades mRNA. The corresponding Hades siRNA sequences are, the former, 5'-GCCUCGAGAUAAAAAUCCCtg-3'(antisense sequence: SEQ ID NO: 4) and 5'-GGGAUUUUUAUCUCGAGGCtt-3'(sense sequence); and, the later, 5'-UUUGUCCUCUACACACACGtg-3'(antisense sequence: SEQ ID NO: 5) and 5'-CGUGUGUGUAGAGGACAAAtt-3'(sense sequence). Among them, the strand capable to bind to the taget gene sequence is expressed as an anti-sense strand, and the other strand is expressed as a sense strand. In ordet to inject the siRNA into the cell, the siRNA should be synthesized as a form of double strand, if not the cell cannot recognize the siRNA.

The sense and antisense strands of the siRNA of the present invention may include two complementary, single-stranded RNA molecules, or a molecule wherein two complementary moieties are base-paired and covalently bonded by a single-stranded "hairpin" domain. The latter is called shRNA (short hairpin RNA). shRNA is a single strand, about 50-70 nucleotides in length, having a stem-loop structure in vivo. On both sides of 5-10 nucleotide loop portion, long RNA of 19-29 nucleotides are base-paired to form a double-stranded stem. In general, shRNA is synthesized in vivo from the Pol III promoter by the transcription of complementary DNA sequence. The Pol-III-induced transcription starts from the well-defined start site and terminates at the linear second residue consisting of 4 or more thymidines (-TTTT-) to form a non-poly(A) transcript. The Pol III promoter is activated in all cells and can express the shRNA. Following the transcription, the shRNA has its loop cleaved by Dicer, and interacts with RISC like siRNA [see Tuschl et al., Cell 110(5): 563-74 (2002)].

The siRNA of the present invention may be obtained by the method well known to those skilled in the related art. For example, the siRNA may be synthesized chemically or produced by recombinant technique using the method well known in the related art. Preferably, the siRNA of the present invention may be synthesized chemically using adequately protected ribonucleoside phosphoramidites and a commonly used DNA/RNA synthesizer. The siRNA may be synthesized as two separated complementary RNA molecules or as an RNA molecule having two complementary domains. Alternatively, the siRNA may be expressed from a recombinant DNA plasmid using an adequate promoter. Examples of the adequate promoter for expressing the siRNA of the present invention from plasmid may include U6 or H1 RNA pol III promoter and cytomegalovirus promoter. Further, the recombinant plasmid may include an inducing promoter or a controllable promoter so that the siRNA can be expressed under a specific tissue or cell environment.

The siRNA of the present invention may be expressed from the recombinant plasmid as two separated complementary RNA molecules or as an RNA molecule having two complementary domains. Selection of adequate plasmid for expressing the siRNA of the present invention, insertion of nucleotide sequence for expressing the siRNA into the plasmid, and transfer of the recombinant plasmid to target cells are disclosed in the related art. For example, refer to the literatures [Tuschl et al., Nat. Biotechnol., 20: 446-448 (2002); Brummelkamp et al. Science 296: 550- 553 (2002); Miyagishi et al., Nat. Biotechnol. 20: 497-500 (2002); Paddison et al., Genes Dev. 16: 948-958 (2002); Lee et al., Nat. Biotechnol. 20: 500-505 (2002); and Paul et al., Nat. Biotechnol. 20: 505-508 (2002)], which are incorporated herein by reference.

In the composition for suppressing tumor of the present invention, the expression inhibitor of Hades protein may be Hades siRNA (short interfering RNA) having a base sequence complementary to the mRNA of Hades gene, more perferably a base sequence of SEQ ID NO: 4 or 5, or a gene transcribing the Hades siRNA.

In the composition for suppressing tumor of the present invention, the action inhibitor of Hades protein may be a substance that inhibits the interaction between Hades and p53. Examples of the substance that inhibits the interaction between Hades and p53 may include anti-Hades antibody, etc. which can bind specifically to the p53-binding site (RNIG-finger domain at the C-terminus) of Hades.

In the composition for suppressing tumor of the present invention, the interaction between Hades and p53 inhibits DNA binding of p53, transcription activity, stress-induced apoptosis and p53-induced apoptosis.

The present invention elucidates that the interaction between Hades and p53 prevents functions of p53 described above. The term used herein "functions of p53" refers to antitumoric effects of p53 generally known to one of skill in the art including not only inhibition of tumorigenesis by arresting the cell cyle at G1 phase to repair DNA damages upon intracellular DNA damages but also inhibition of cancer development by inducing apoptosis of abnormal cells through a programmed cell death (PCD) mechanim upon abrupt proliferation of cancer cells.

In the composition for suppressing tumor of the present invention, the interaction betweem Hades and p53 induces ubiquitination of p53.

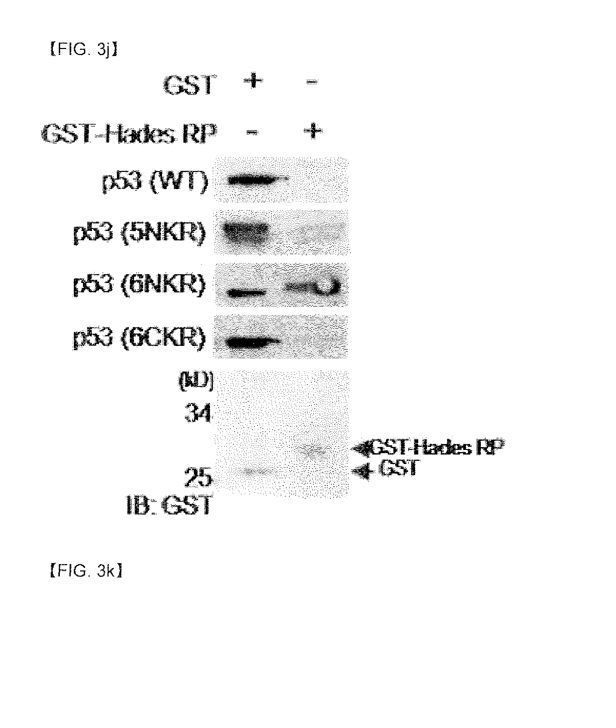

The present inventors have discovered that the interaction betweem Hades and p53 occurs in cytosol and induces degradation of cytoplasmic p53, particulary by causing ubiquitination at the 24^{th} residue, a lysine residue in the N-terminal portion of p53 (see Fig. 3j).

In the composition for suppressing tumor of the present invention, the inhibitors to expression or action of Hades targets to the RNIG-finger domain at the C-terminus of Hades.

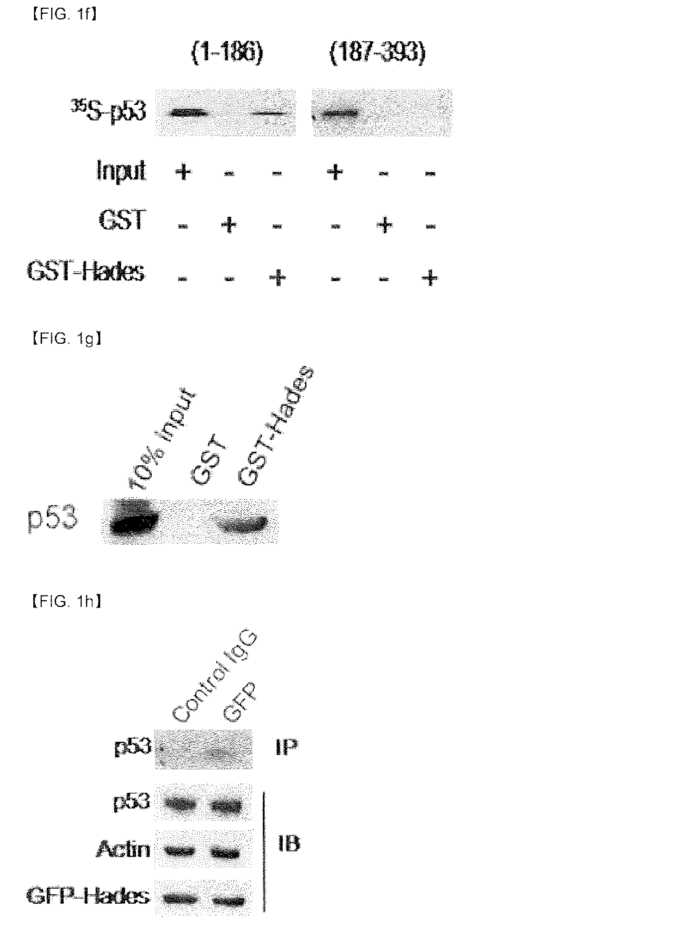

The present inventors have discovered that the RNIG-finger domain at the C-terminus of Hades binds to the DNA-binding domain of p53 (see Fig. 1f). Therefore, peptides may be designed for competitive interfering the interaction between Hades and p53, which are specifically bound to the p53 binding site of Hades.

According to another embodiment of the present invention, there is provided a method for screening anti-cancer agent comprising:
(a) treating an agent in animal cells expressing Hades protein; and
(b) determining whether the expression of Hades protein decreases as compared to a control group in which the animal cells are not treated with the agent.

In the step (a), the animal cells may be intentionally transfected with a plasmid overexpressing Hades. In the step (b), the decrease of expression may be measured by RT-PCR in RNA level, or by Western blotting, etc. in protein level.

According to another embodiment of the present invention, there is provided a method for screening anti-cancer agent comprising:
(a) contacting Hades protein comprising p53-binding site or its fragment and p53 in the existance of an agent; and
(b) determining whether the interaction between Hades and p53 is inhibited as compared to a control group in which the agent does not exist.

The term used herein "anticancer agent" refers to substances capable of preventing or treating cancers by suppressing the inhibitory effects of Hades to p53.

The term used herein "inhibition of the interaction" refers to inhibition or elimination of the interaction between Hades and p53, including inhibitions by suppressing the interaction *per se* between Hades and p53, or each of Hades and p53.

In the Method for screening anti-cancer agent of the present invention, the fragment of Hades comprises p53-binding site (RING-finger domain at the C-terminus) having an amino acid sequence of SEQ ID NO: 3.

In the Method for screening anti-cancer agent of the present invention, the interaction between Hades and p53 at the step (b) occures between p53-binding site (RING-finger domain at the C-terminus)of Hades and DNA-bindg domain of p53.

In the Method for screening anti-cancer agent of the present invention, the interaction between Hades and p53 at the (b) step may be detected by any conventional method known in the art, preferably by immunoprecipitation assay, GST pull-down assay and colony-formation assay.

The "immunoprecipitation assay" is one of the most prevalent immnochemical technologies using affinity between antigens and antibodies to specifically isolate antigens (or affinity materials to antigens). The technology is generally used to analyze antigen molecular weights, protein-protein interaction, enzyme activities, post-transcriptional modifications of proteins and amounts and existence of proteins. The immunoprecipitation analysis enables to detect proteins in very low levels by concentrating proteins upto 10,0000-fold. In the immunoprecipitation analysis, a protein is extracted from cells or tissues using a lysis buffer and the bound to a primary antibody and Protein A-, G- or L-agarose, or a secondary antibody-agarose. The binding to the secondary antibody-agarose ensures separation of other proteins not bound to antibodies to target proteins. The selection of agarose is dependent on isotype and species origin of primary antibodies. In addition, the immunoprecipitation assay is employed to detect proteins binding to a protein of interest under non-denaturing conditions.

The "GST pull-down assay" is a technology to test interactions between either a tagged protein the the bait (e.g., GST, His₆ and biotin) and other protein (test protein or prey). The bait purified in a suitable expression system (e.g., *Escherichia coli*) is immobilized on a glutathione affinity gel. The bait is used as a secondary affinity support to discriminate a new protein partner or verify protein estimated as a protein partner.

The "colony-formation assay" is a technology to anayze and test cell growh potentials by observing colony formation of a cell. The colony is defined to comprise 50 cells. Cance cells have indefinite proliferation potential. The inhibitory effects of a gene against cancer cell proliferation may be evaluated by transformation of the gene into cancer cells and culturing to observe formation and number of colonies.

According to another embodiment of the present invention, there is provided an anti-cancer composition comprising the agent screened by the method for screening anti-cancer agent of the present invention as an effective ingredient.

The anti-cancer composition of the present invention may comprise any substance capble of inhibiting interaction between Hades and p53, in particular, substance capble of inhibiting or eliminating the interaction *per se* between Hades and p53, or each of Hades and p53.

In the pharmaceutical compositions of this invention, the pharmaceuticaily acceptable carrier may be conventional one for formulation, including lactose, dextrose, sucrose, sorbitol, mannitol, starch, gum acacia, calcium phosphate, alginate, gelatin, calcium silicate, microcrystalline cellulose, polyvinylpyrrolidone, water, salt solutions, alcohols, gum arabic, syrup, methyl cellulose, methylhydroxy benzoate, propylhydroxy benzoate, talc, magnesium stearate and mineral oil, but not limited to. The pharmaceutical compositions of this invention, further may contain lubricant, wetting agent, sweetening agent, flavors, emulsifier, suspending agent and preservatives.

It is prefered that the pharmaceutical composition of this invention may be administered parenterally, for examp, by intravenous injection, intraperitoneal injection, intratumoric injection, intramuscular injection, subcutaneous injection, intra-cardial muscular injection or local injection. For example, the pharmaceutical composition may be administered intraperitoneally to treat ovarian cancer and intravenously to treat liver cancer, directly injected to visible tumor mass to treat breast cancer, directly injected to enema to treat colon cancer, and directly injected to a catheter to treat bladder cancer.

The correct dosage of the pharmaceutical compositions of this invention will be varied according to the particular formulation, the mode of application, age, body weight and sex of the patient, diet, time of administration, condition of the patient, drug combinations, reaction sensitivities and severity of the disease. It is understood that the ordinary skilled physician will readily be able to determine and prescribe a correct dosage of this pharmaceutical compositions. According to a preferred embodiment of this invention, where the pharmaceutical composition comprising siRNA is administered, a suitable dosage is 2-5 mg/kg. In using Hades inhibitors, a preferable dosage is 0.1-1 g/kg. It is important that the present pharmaceutical composition is administered in the amounts to achieve maximum efficacies with the minimum dosage considering the factors, which may be determined by one of skill in the art.

According to the conventional techniques known to those skilled in the art, the pharmaceutical compositions of this invention can be formulated with pharmaceutical acceptable carrier and/or vehicle as described above, finally providing several forms including a unit dosage form. Non-limiting examples of the formulations include, but not limited to, a solution, a suspension or an emulsion, an extract, an elixir, a powder, a granule, a tablet, a capsule, emplastra, a liniment, a lotion and an ointment.

The pharmaceutical compostion of the present invention may be solely administered or in a combination with conventional cheomtherpies or radiotherapy, including cisplatin, carboplatin, procarbazine, mechlorethamine, cyclophosphamide, ifosfamide, melphalan, chlorambucil, bisulfan, nitrosourea, ciactinomycin, daunorubicin, doxorubicin, bleomycin, plicomycin, mitomycin, etoposide, tamoxifen, taxol, transplatinum, 5-fluorouracil, vincristin, vinblastin and methotrexate.

According to another embodiment of the present invention, there is provided a kit for screening anti-cancer agent which inhibits interaction between Hades and p53 comprising:
(a) a polypeptide comprising p53-binding site (RING-finger domain at the C-terminus)of Hades;
(a) a polypeptide comprising DNA-binding domain of p53; and
(c) an agent for detecting the interaction between the polypeptides.

Preferably, the screening method of this invention may further comprise the step of selecting druggable anti-cancer agents by measuring the binding affinity of test substances to the p53-binding site of Hades before the step (a). Therefore, the kit of this invention may be employed to embody the screening method of this invention.

According to another embodiment of the present invention, there is provided a use of an expression or action inhibitor of Hades protein having an amino acid sequence of SEQ ID NO:2 for manufacturing an antitumoric composition.

According to another embodiment of the present invention, there is provided a method for suppressing tumor, comprising administering to a subject in need thereof a pharmaceutically effective amount of an expression or action inhibitor of Hades protein comprising an amino acid sequence of SEQ ID NO:2.

### [Advantageous Effects]

The present inventors have found that the overexpressed Hades protein interacts with p53 to inhibit the exonuclear mechanism of p53 and the knowdown of Hades induces increase in the expression of p53, demonstrating that Hades is a negative regulator to p53. Therefore, it would be understood that the inhibition of Hades overexpressed in tumor cells contributes to tumor-supressive effects of p53.

### [Description of Drawings]

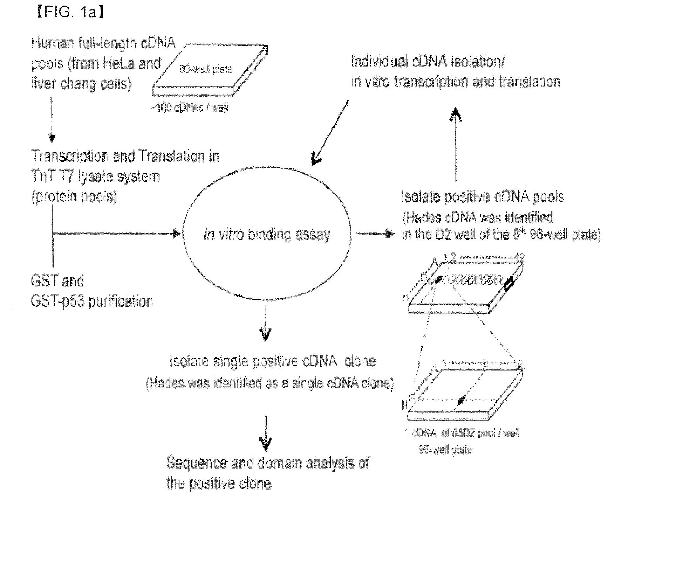

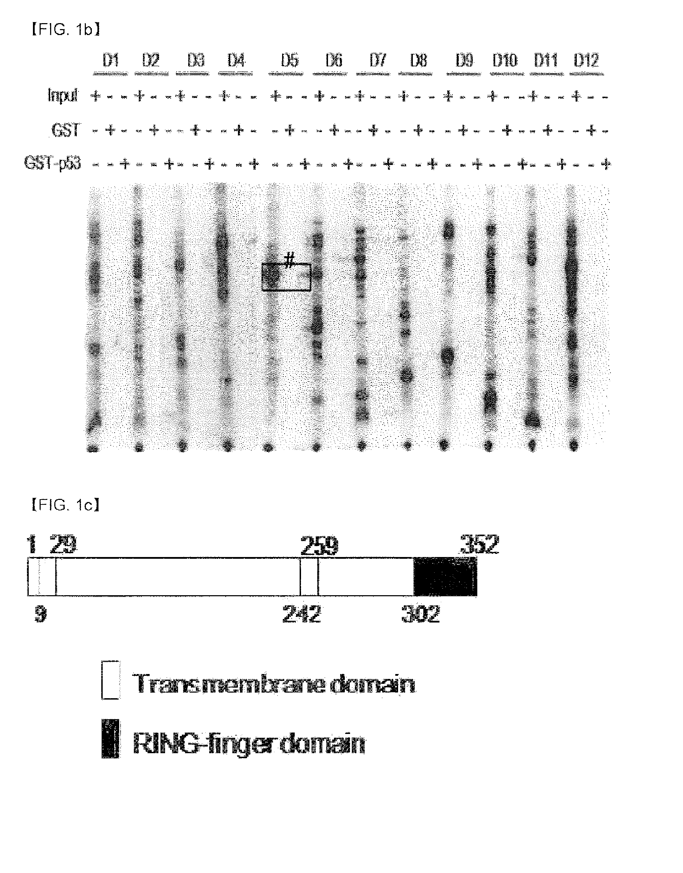

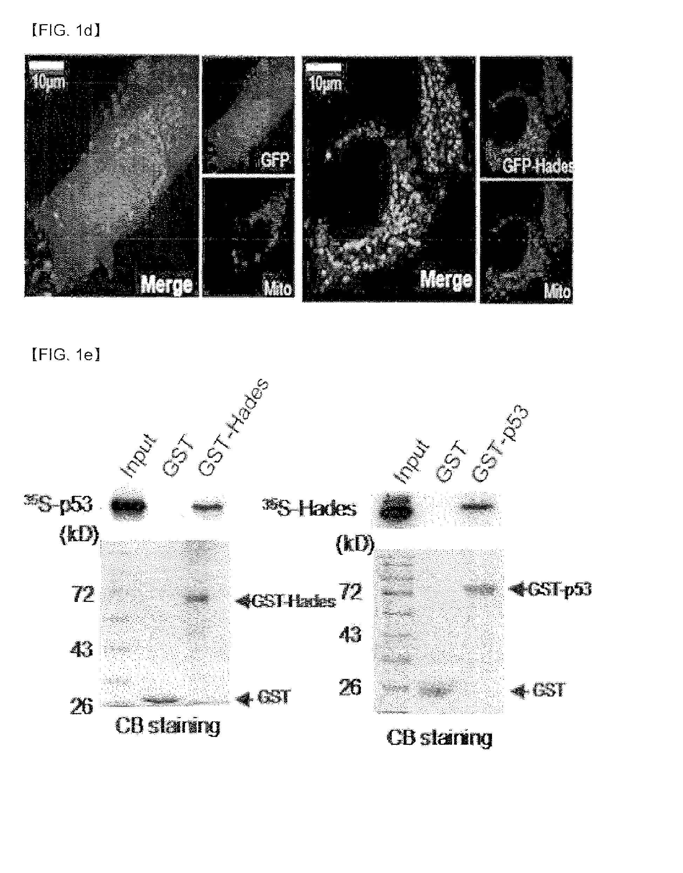

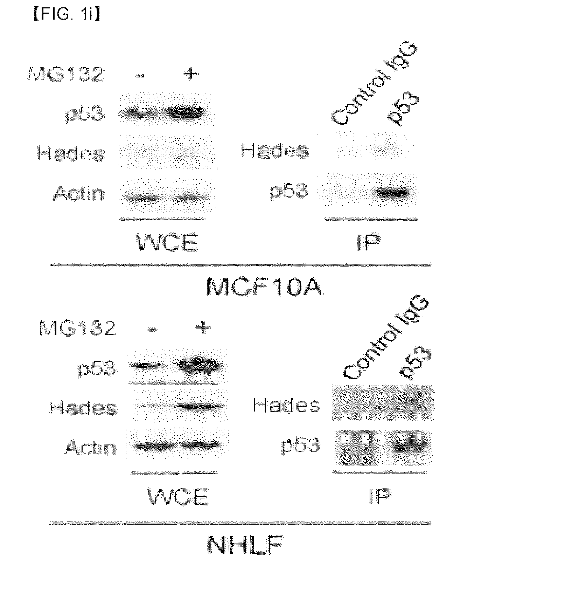

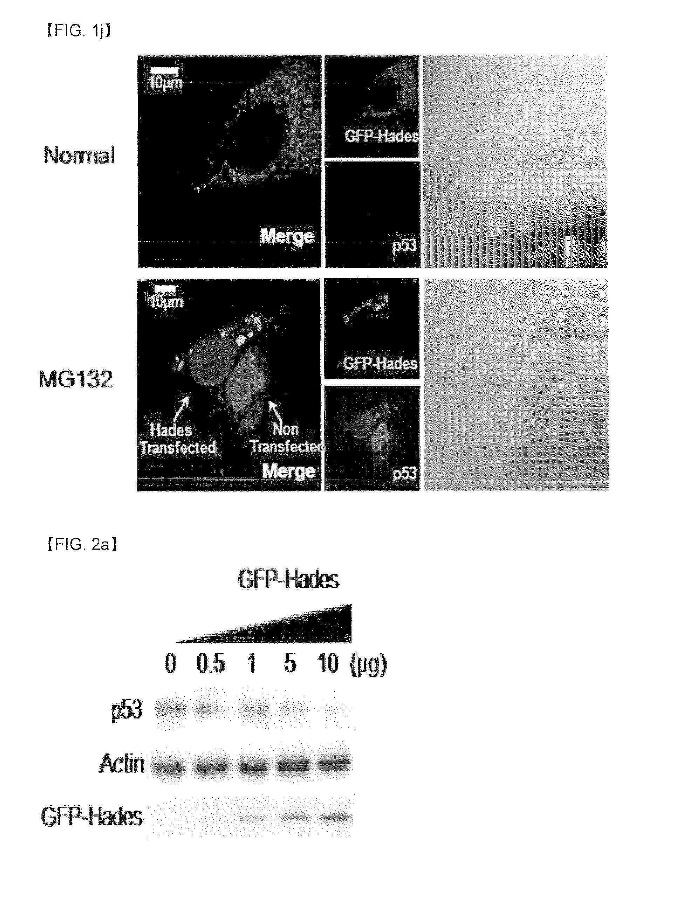

Fig. 1a shows a schematic diagram of the screening procedure used for identifying novel p53 interacting partners; Fig. 1b shows *in vitro* transfected ³⁵S-labeled proteins interacting with p53 using cDNA library; Fig. 1c shows domain of Hades; Fig. 1d shows confocal microscopic images showing Hades localization in U2OS cells. control GFP (upper panel) or GFP-Hades (lower panel) plasmid; Fig. 1e shows *In vitro* pull down assay showing that Hades interacts with p53; Fig. 1f shows a result of measuring the interaction between N- or C-terminal region and p53 for investigation of p53 binding site of Hades; Fig. 1g shows the interaction between Hades and p53 with *in vitro* pull-down assays; Fig. 1h shows the interaction between Hades and p53 in H1299 cells *in vivo* by immunoprecipitation; Fig. 1i shows the interaction between Hades and p53 in MCF10A and NHLF cells *in vivo* by immunoprecipitation; and Fig. 1j shows colocalization of Hades and p53 through confocal microscopy.

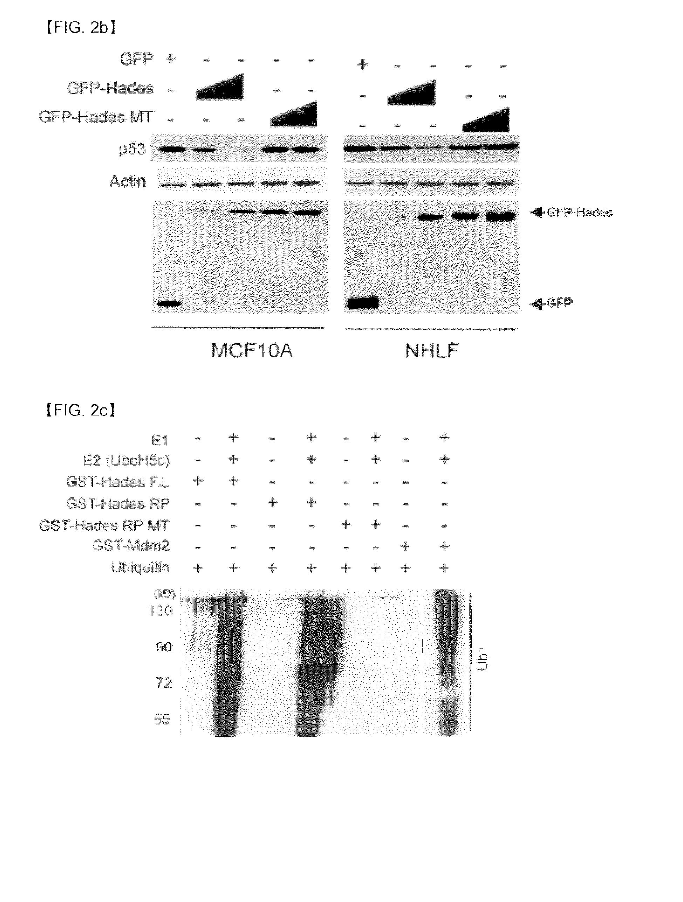

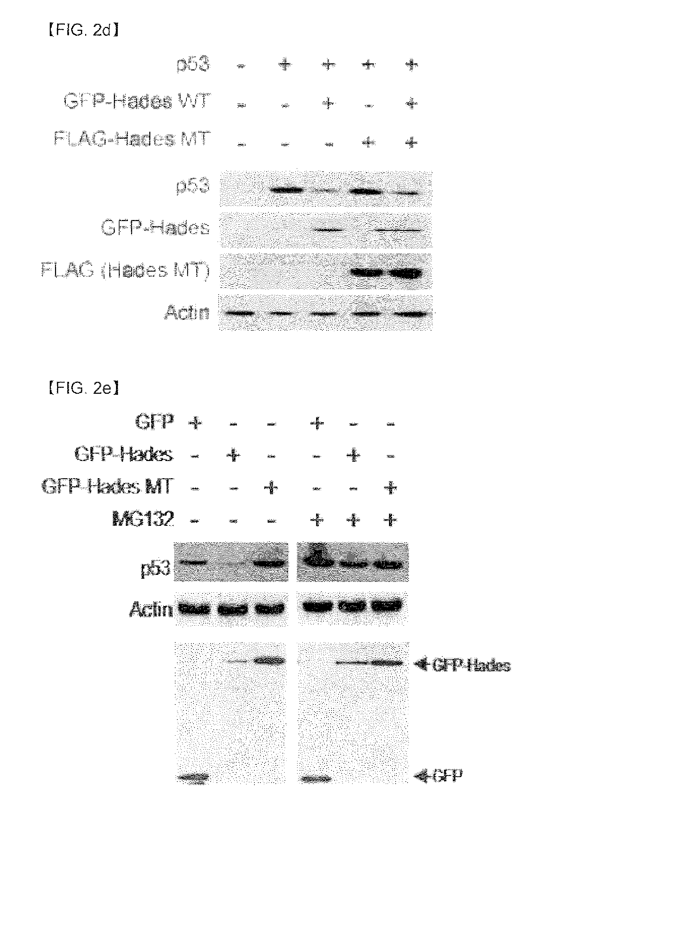

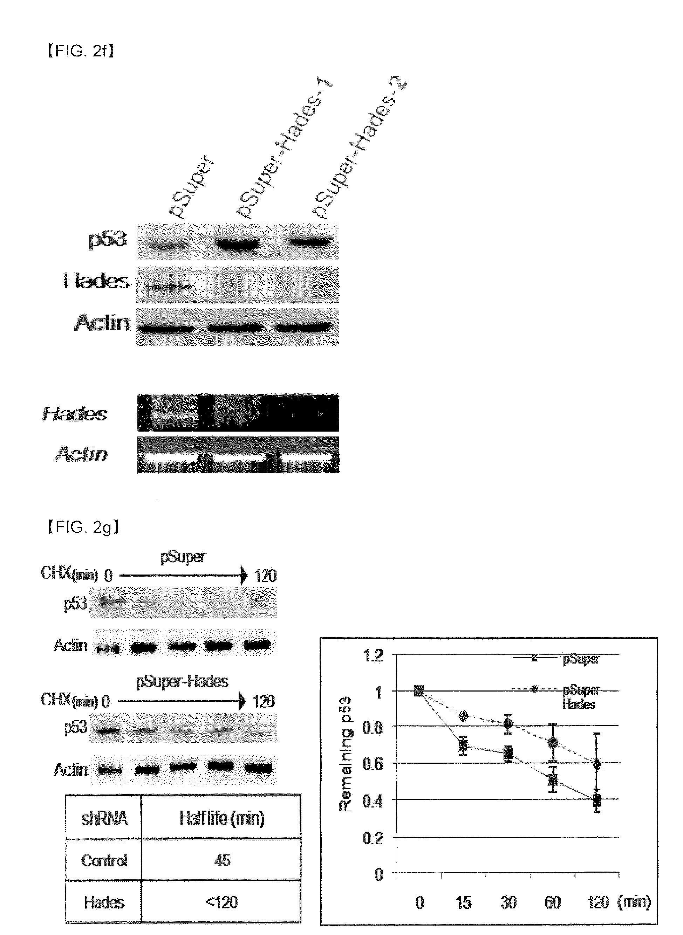

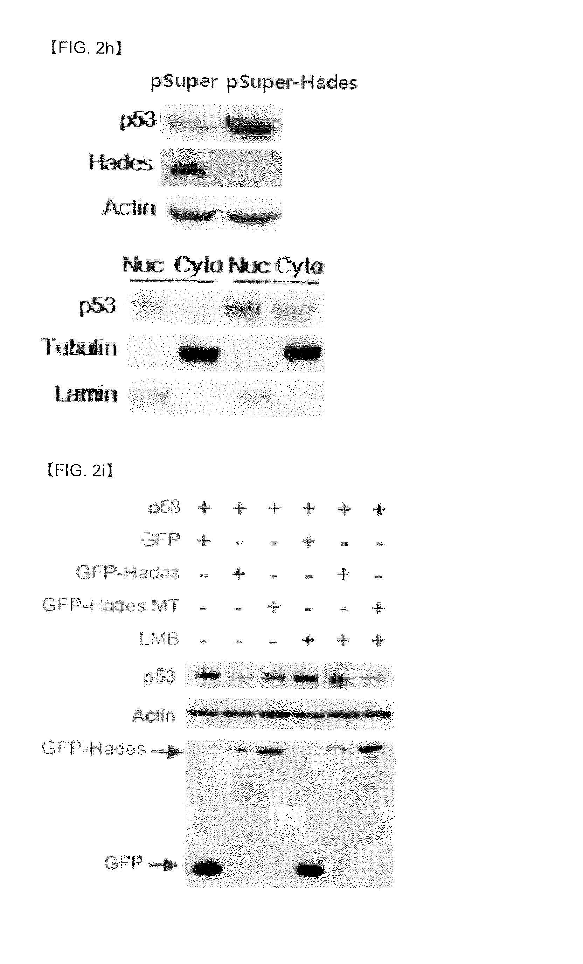

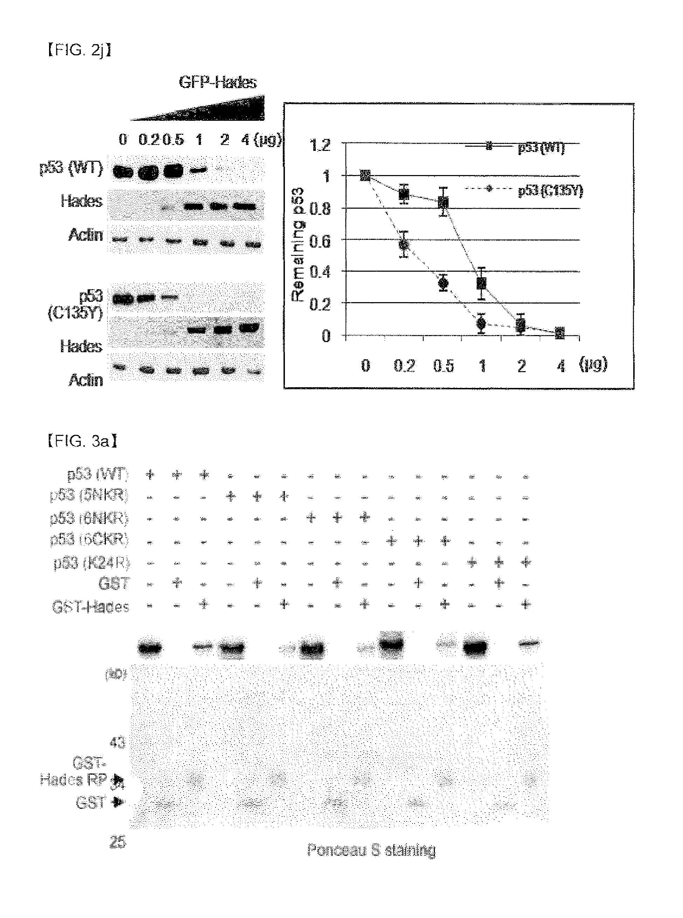

Fig. 2a shows change of level of p53 protein for levels of Hades; Fig. 2b shows change of level of p53 protein for levels of Hades in MCF10A and NHLF cells; Fig. 2c shows autoubiquitination activities of Hades; Fig. 2d shows reduced p53 activity of RIN-finger domain of Hades; Fig. 2e shows that the reduced level of p53 by Hades was recovered by treatment of MG132; Fig. 2f shows that level of p53 was recovered by silencing of Hades usign Hades shRNA; Fig. 2g shows kinetic analysis on stablization of p53 by Hades silencing in the cells treated with CHX; Fig. 2h shows level of p53 protein by knockdown of Hades in the nucleus and cytoplasm; Fig. 2i shows that p53 reduced by Hades was recovered by LMB treatment; Fig. 2j shows level of p53 protein in the WT p53 and mutant p53 for expression of Hades.

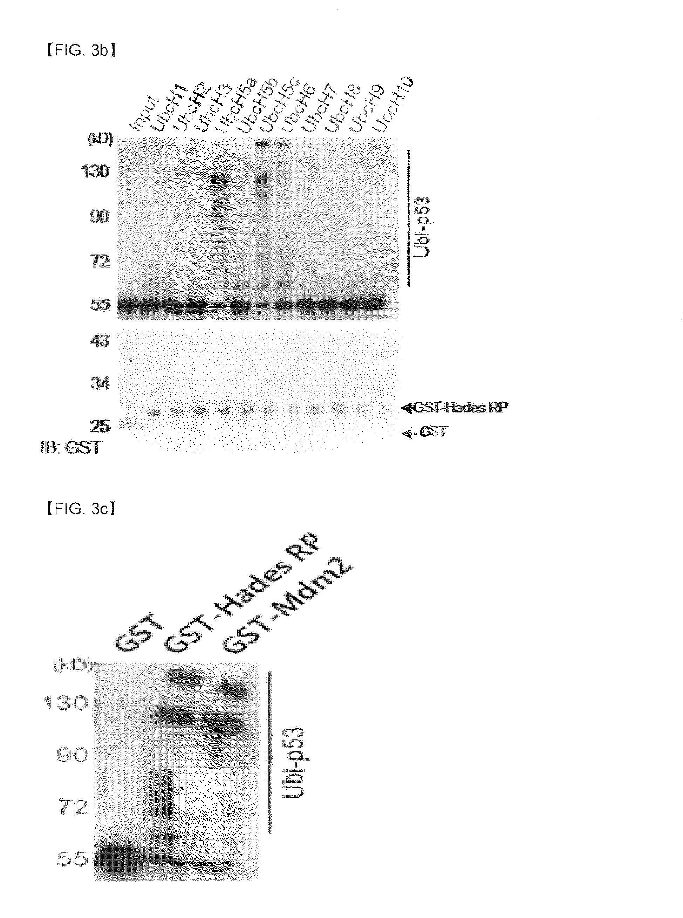

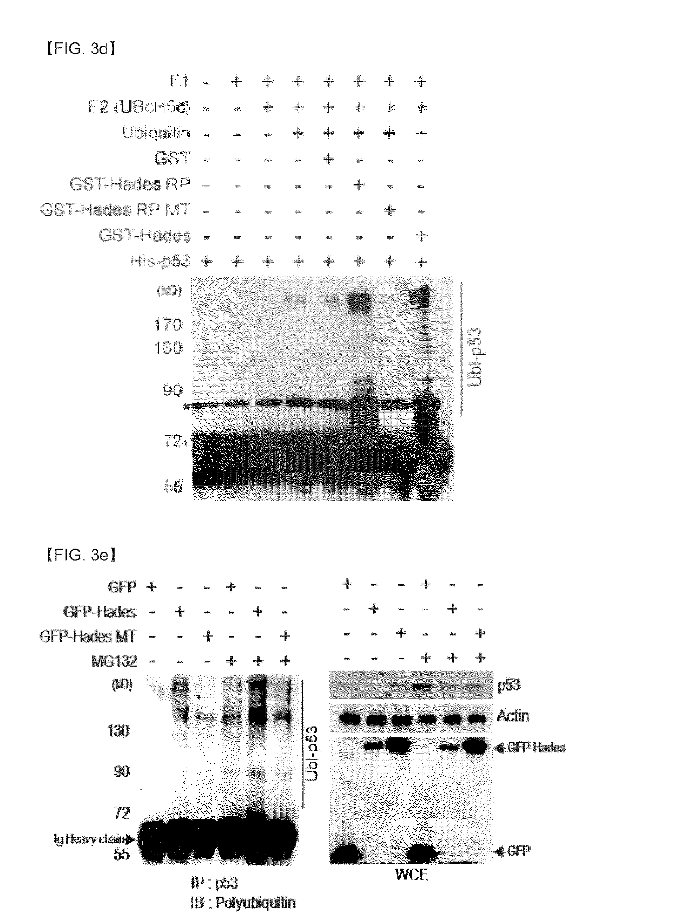

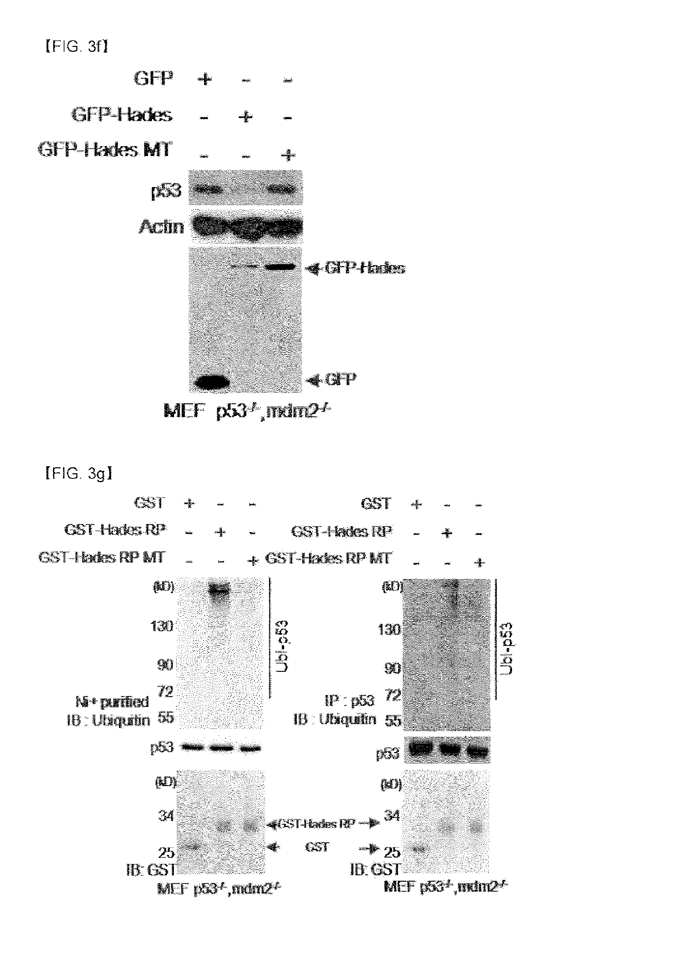

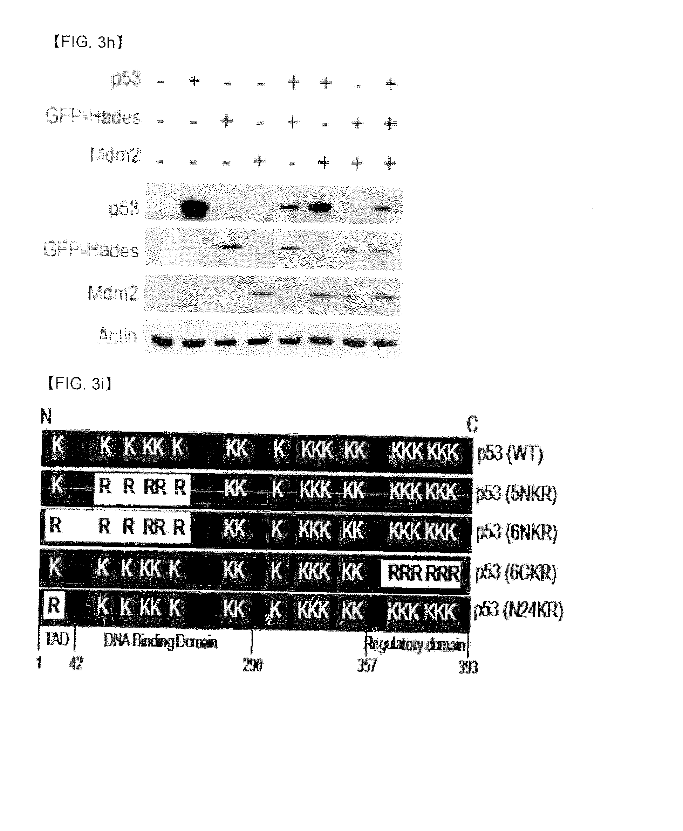

Fig. 3a shows that Hades interacted with mutant p53; Fig. 3b shows that Hades ubiquitinated p53; Fig. 3c shows that Hades or Mdm2-mediated ubiquitination of p53 was evaluated by *in vitro* ubiquitination assays; Fig. 3d shows that Hades-induced ubiquitination of p53 was evaluated by *in vitro* ubiquitination assays and immunoblotting; Fig. 3e shows that Hades-induced ubiquitination of p53 was evaluated by *in vitro* ubiquitination assays; Fig. 3f shows that degradation of p53 by Hades was not dependent on Mdm2; Fig. 3g shows ubiquitination of p53 by Hades in the presence of GST-Hades RP in MEF *p53*^{*-*/}*⁻ mdm2*^{*-*/*-*} cells; Fig. 3h shows that Mdm2 does not affect on ubiquitination of p53 by Hades; Fig. 3i shows whether p53 lysine residue affect on ubiquitination of p53 by Hades; Fig. 3j shows that N-terminal lysine 24 lysine residue of p53 affect on ubiquitination of p53 by Hades; and Fig. 3k shows that mutation p53 K24R does not affect on ubiquitination of p53 by Hades.

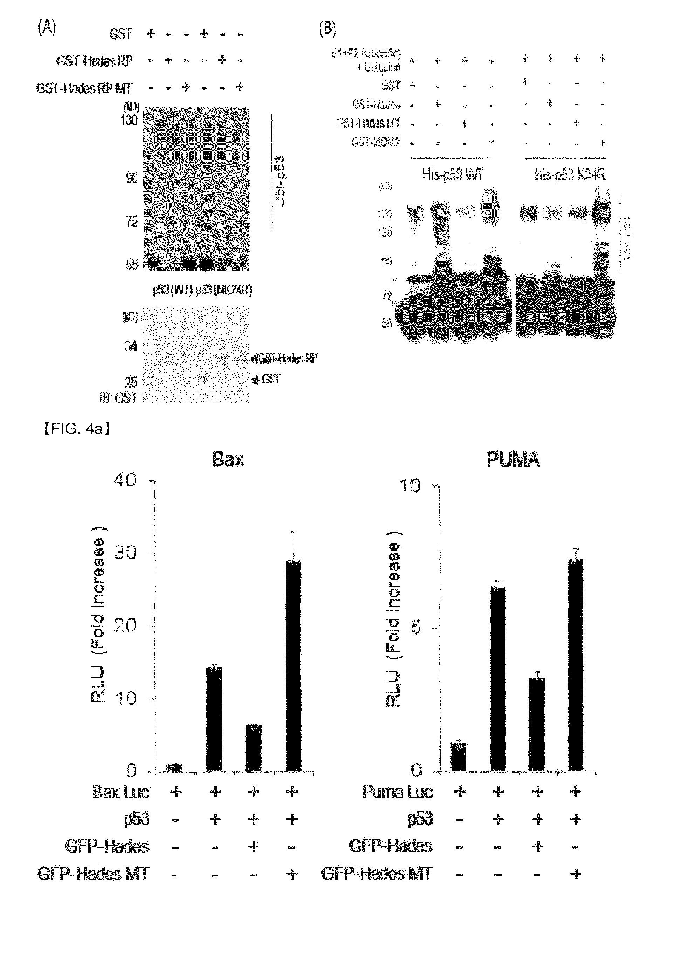

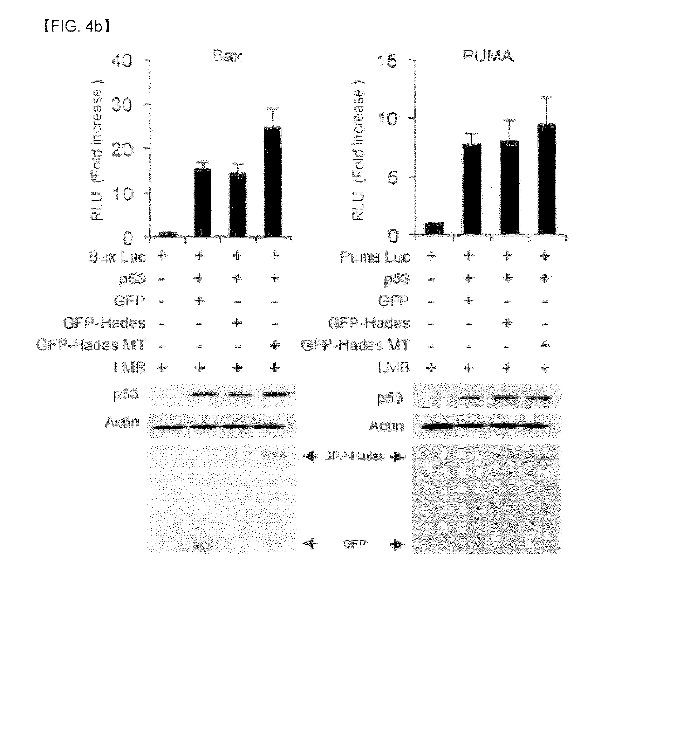

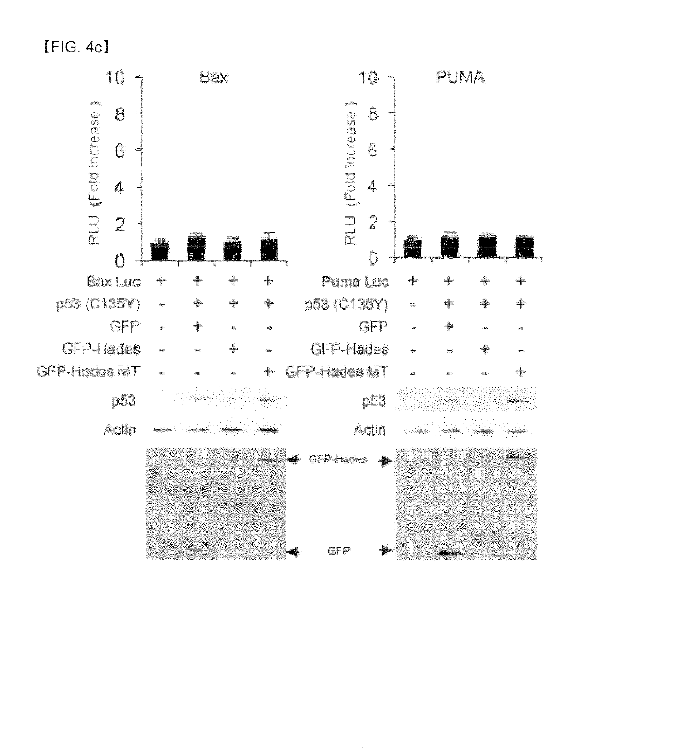

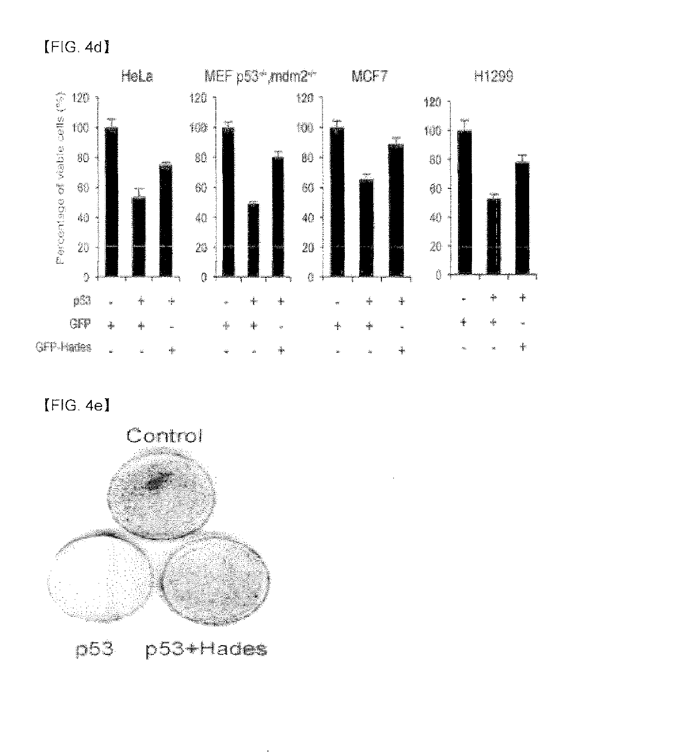

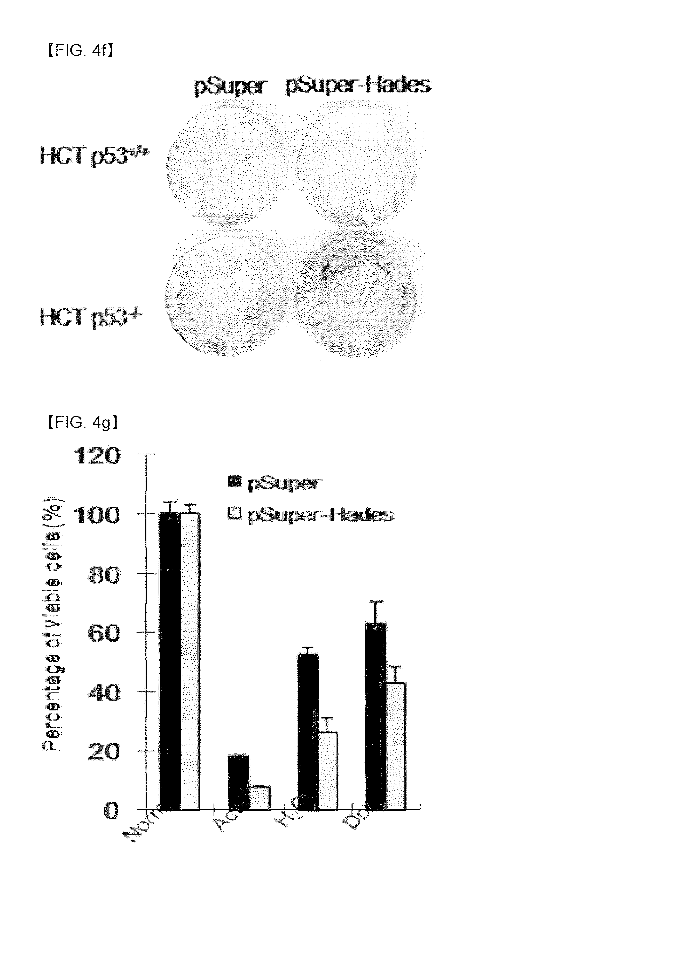

Fig. 4a shows that p53-dependent transcriptional activity was suppressesed by Hades; Fig. 4b shows that p53-dependent transcriptional activity was not supperssesed by hades in LMB(leptomycin B); Fig. 4c shows Hades-mediated degradation of a transactivation-deficient mutant of p53 showing that p53 degradation by Hades is not related with transcriptional activity; Fig. 4d shows that overexpressed Hades inhibits p53-mediated growth suppression; Fig. 4e shows that Hades inhibits cell growth suppression of p53 by colony formation assays; Fig. 4f shows that Hades-knockdown induces recovery of p53 by colony formation assay; and Fig. 4g shows that Hades-konckdown inhibits cell viability in various stress condition.

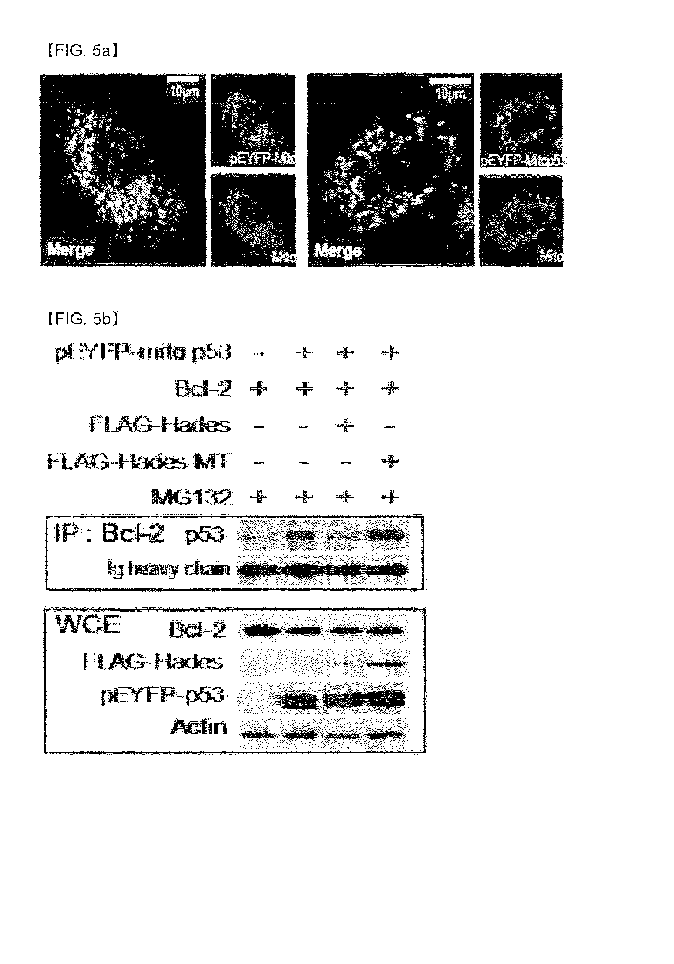

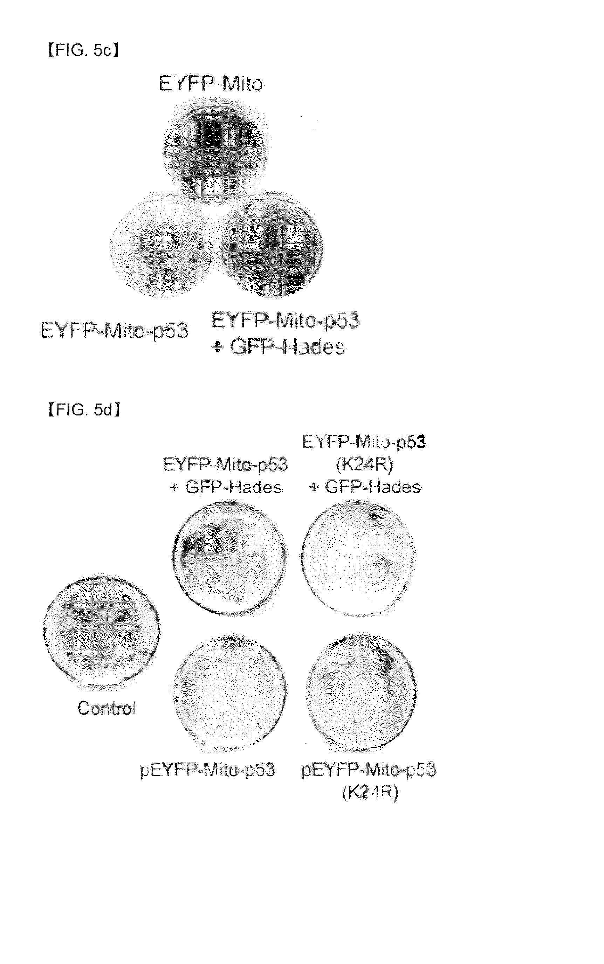

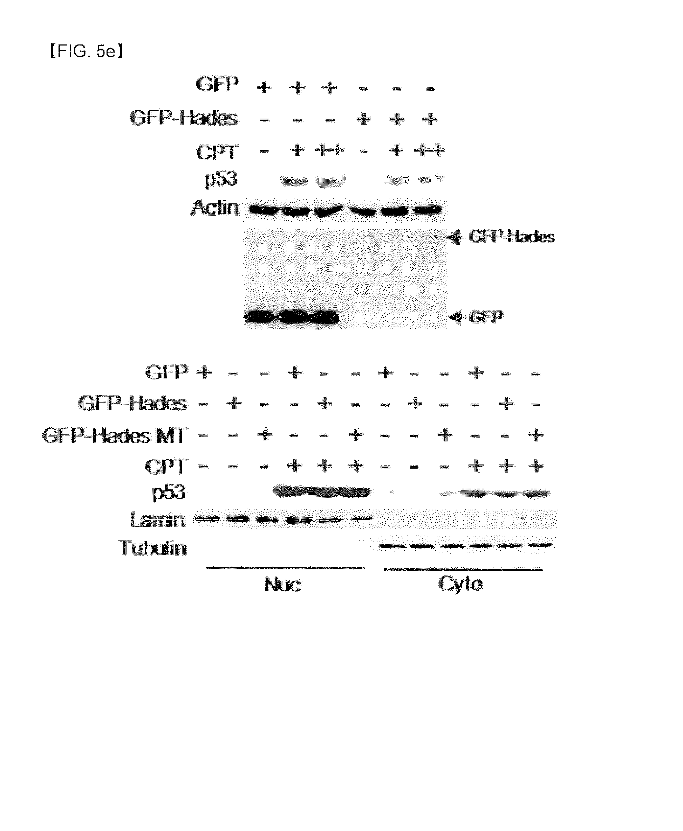

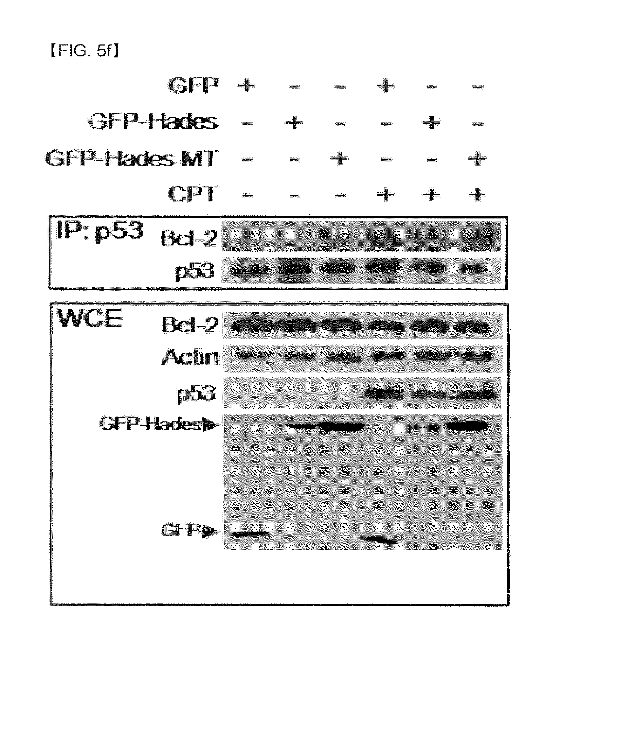

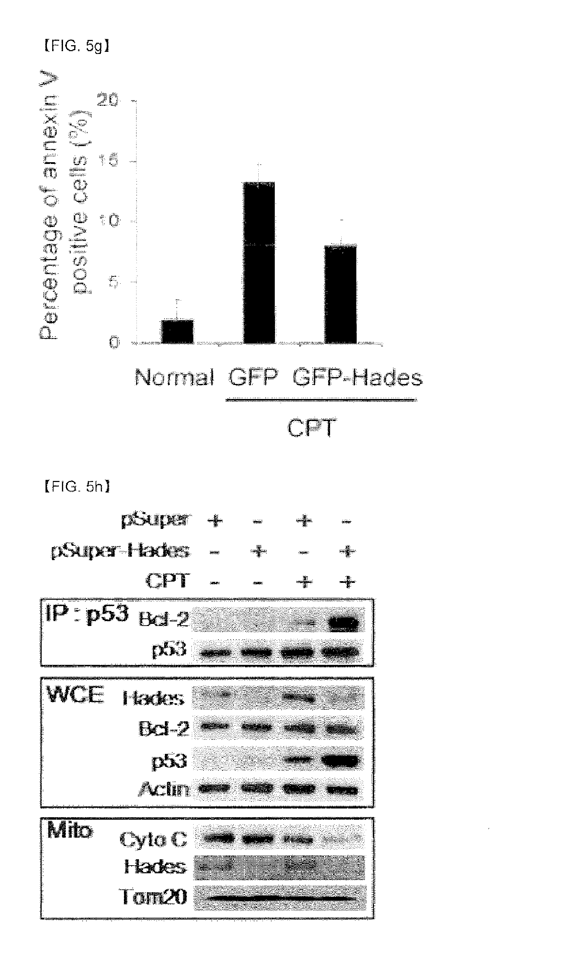

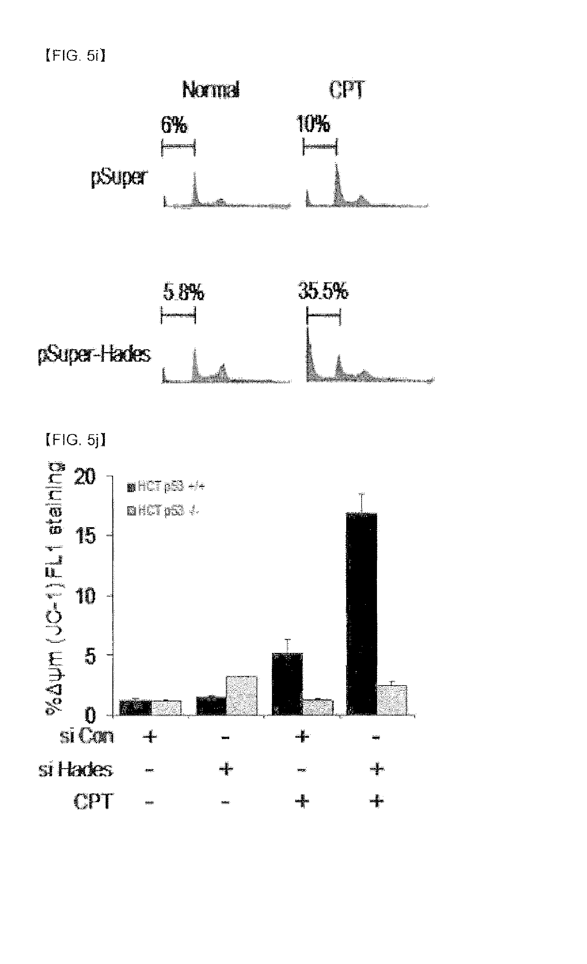

Fig. 5a shows that pEYFP-mito-p53 is localized in the mitochondria in U2OS cells transfected with pEYFP-mito-p53; Fig. 5b shows that interaction between pEYFP-mito-p53 and Bcl-2 was inhibited by ectopically expressed Hades; Fig. 5c shows that colony formation in H1299 cells transfected with pEYFP-mito-p53 was inhibited by ectopically expressed Hades; Fig. 5d shows that the ectopically expressed Hades does not inhibit colony formation in MEF cell(p53-/-;Mdm2-/-) transfected with mutant pEYFP-mito-p53(K24R) by colony formation assays; Fig. 5e shows that Hades decreases p53 expression level induced by CTP; Fig. 5f shows that Hades inhibits the interaction between p53 and Bcl-2 in CPT-treated cells; Fig. 5g shows that CPT-induced apoptosis was suppressed by Hades; Fig. 5h shows that Hades-knockdown stimulates the interation between Bcl-2 and p53 in CPT-treated cells; Fig. 5i shows that Hades-knockdown leads to increase susceptibility of apoptosis in CPT-treated cells; Fig. 5j shows that CPT-induced knockdown of Hades stimulates CPT-induced mitochondrial damage; and Fig. 5k shows a result of measuring the hades expression level in HCC (human primary hepatocellular carcinoma) tissues and adjacent normal tissues

### [Best Mode]

Practical and presently preferred embodiments of the present invention are illustrated as shown in the following Examples. However, the present invention is not restricted to the following Examples, and many variations are possible within the spirit and scope of the present invention.

### EXAMPLE 1. Reagents and Methods

### 1-1. Cell culture and transfection

MCF7, A549, U2OS, and HeLa cells were purchased from the Korean Cell Line Bank (Korea). MEF p53^{-/-} and MEF p53^{-/-}; Mdm2^{-/-} cells were kind gifts from Dr. Wei Gu (Columbia University, USA). MCF7, 293, 293T, HCT116 p53^{+/+}, HCT116 p58^{-/-} cells MEF p53^{-/-}, and p53^{-/-}, Mdm2^{-/-} cells were maintained in DMEM media with 10% fetal bovine serum (FBS) and 1% penicillin/streptomycin. U2OS and H1299 cells were maintained in RPMI media with 10% FBS and 1% penicillin/streptomycin. Stable pSuper-Hades MCF7 cells were maintained in DMEM supplemented with 10% FBS and 500 µg/ml G418 (neomycin). Transient transfections were performed using Hilymax (Dojindo, Japan) according to the manufacturer's instructions. For siRNA transfection, RNAimax (Invitrogen) was used according to the manufacturer's instructions. siRNA for Hades and mock siRNA were obtained from Ambion (USA) and had the following sequences: Hades siRNAs, GGGAUUUUUAUCUCGAGGC and CGUGUGUGUAGAGGACAAA; mock siRNA, AUGAACGUGAAUUGCUCAAG.

### 1-2. Antibodies and reagents

Mouse monoclonal antibodies against p53 (Do-1), tubulin (B7), Bcl-2 (C2), and ubiquitin (P4D1); goat polyclonal antibodies against lamin (C20); anti-goat IgG-HRP (sc-2020); normal mouse IgG (sc-2025); anti-mouse IgG-Texas Red (sc-2781); and protein A agarose (sc-2001) and protein G agarose (sc-2002) were purchased from Santa Cruz Technologies (USA). Rabbit polyclonal antibody against Hades was generated by Labfrontier (Korea) using peptide sequences N-SGERPKGIQETEEM-C and N-SRAKPEDRESLKSAC-C. Anti-β-actin antibody (A5441) was purchased from Sigma; anti-mouse IgG-HRP (7076) and anti-rabbit IgG-HRP (7074) were purchased from Cell Signaling Technology (USA)); anti-cytochrome C (556433), and Tom 20 (612278) antibodies were from BD Pharmingen (USA); and polyubiquitin chain (FK-1) antibody was from BioMol (USA). For *in vitro* ubiquitination, assays, E1 (Ube1), E2 (UbcH1, UbcH2, UbcH3. UbcH5a, Ubc5Hb, Ubc5Hc, UbcH6, UbcH7, UbcH8, UbcH9, UbcH10), and His-ubiquitin were purchased from Boston Biochem (USA). Camptothecin (CPT), Actinomycin D, crystal violet, propidium iodine, and DAPI were purchased from Sigma, cycloheximide from Biopure (Canada), and MG132 (474791) from Calbiochem (USA). The JC-1 staining kit and Nutlin 3 were purchased from Cayman Chemical (USA). Annexin V was purchased from BD Pharmingen and Leptomycin B from Alexis Biochemical (USA).

### 1-3. Production of Plasmids

Hades gene was isolated from HeLa cell cDNA by RT-PCR using the primers indicated and subcloned into the following vectors

**[Table 1]**

| Vetors and primers | | |
|---|---|---|
| Vectors | Primer | |
| pCITE4 (Novagen) | forward | GGAATTCCATGGAGAGCGGAGGC |
| | reverse | GGAATTCCTTAGCTGTTGTACAGGGTATC |
| pEGX6p3 (Amersham Bioscience) | forward | GGAATTCCATGGAGAGCGGAGGGCGGC |
| | reverse | GGAATTCCTTAGCTGTTGTACAGGGGTATAC |
| pEGFP C1 (Clontch) | forward | GGAATTCCATGGAGAGCGGAGGGCGGC |
| | reverse | CGCGGATCCGCGTTAGCTGTTGTACAG |
| pcDNA3.1 FLAG (Invitrogen) | forward | CGGGATCCCGACCATGGAGAGCGGAGG |
| | reverse | CCGCTCGAGCGTTACTTATCGTCGTCATCC TTGTAATCGCTGTTGTACAGGGG |

To generate the RING inactive mutant (C302S/C305S), the site-directed mutagenesis was performed using pGEX-Hades Ring protein (RP) or pEGFP-Hades and the following primers.

**[Table 2]**

| Primers for point mutations | | |
|---|---|---|
| Inactive mutant | Primer | |
| C302S | forward | TGAAGCTGCTCAGACACACTACAGAGGCGCTCTTCA GACTCTCCCTG |
| | reverse | CAGGGAGAGTCTGAAGAGCGCCTCTGTAGTGTGTCT GAGCAGCTTCA |
| C305S | forward | CTAAAGAGCGCCTCTGTAGTGTCTCTGAGCAGCTTCA AGTCCTGC |
| | reverse | GCAGGACTTGAAGCTGCTCAGAGACAGTACAGAGGC GCTCTTCAG |

To generate the shRNA expression plasmids targeting p53, siRNA oligomers were subcloned into pSuper-GFP-neo vector using the primers shown in Table 3. Where the subcloned shRNA-expressing plasmid is transformed into cells, it produces excessively only shRNA. More specifically, a DNA sequence corresponding to shRNA is cloned into a vector, and the vector is transformed into cells to generate shRNA. The shRNA vectors enable to save synthesis costs for siRNA and to selectively culture cells with shRNA vectors. The reason that the two following primers are used is to more effectively inhibit the expression of Hades compared to cases using one type of primers.

**[Table 3]**

| Primers for preparing shRNA-expression plasmids | | |
|---|---|---|
| | Primer | |
| Hades- 1 | forward | GATCCCCCGUGUGUGUAGAGG ACAAATTCAAGAGAUUU GUCCUCUACACACACGTTTTTT |
| | reverse | GGGGCACACACAUCUCCUGUUUAAGTTCTCTAAACAGGA GAUGUGUGUGCAAAAATTCGA |
| Hades- 2 | forward | GATCCCCGGGAUUUUUAUCUCGAGGCTTCAAGAGAGCC UCGAGAUAAAAAUCCCTTTTT |
| | reverse | GGGCCCUAAAAAUAGAGCUCCGAAGTTCTCTCGGAGCU CUAUUUUUAGGGAAAAATTCGA |

pcDNA HA-p53 plasmids containing fragmented p53 forms (1-185 and 185-393) were kindly provided by Dr. Gerald M. Cohen (Leicester University, UK). pcDNA-p53 Myc/His, pEYFP mito-p53, and pEGX-p53 were subcloned using pcDNA HA-p53 by PCR using the primers shown in Table 4.

**[Table 4]**

| Primers | | |
|---|---|---|
| | Primer | |
| pcDNA-p53 Myc/His | forward | GGAATTCCACCATGGAGGAGCCGCAGT |
| | reverse | GGAATTCCGCGTCTGAGTCAGGCCCTT |
| pEYFP mito-p53 | forward | CGCGGATCCGATGGAGGAGCCGCAG |
| | reverse | CGCGGATCCCGGTCTGAGTCAGGCCC |

The p53 mutant (C135Y) was kindly provided by Dr. Carl G. Maki (University of Chicago, USA), and p53 lysine mutants (N5KR, N6KR, and C6KR) were kind gifts from Dr. Randy Y. C. Poon (Hong Kong University of Science and Technology, Hong Kong). The p53 N24KR mutant was generated by point mutagenesis PCR using pcDNA p53-FLAG (forward: GACCTATGGAGACTACTTCCTG, reverse: CAGGAAGTAGTCTCCATAGGTC). pGL3-Bax reporter plasmid was kindly provided by Dr. Anastasis Stephanou (University College London, UK). pPV-PUMA FLAG2-Luc was from Dr. Bert Vogelstein (Johns Hopkins Medical Institutions, USA). pCl-Bcl-2 was from Dr. Hiroyuki Osada (Discovery Research Institute, RIKEN, Japan).

### 1-4. Construction of a human full-length cDNA library

Total RNA was isolated from HeLa and liver Chang cells using TRIZOL reagent (Invitrogen, USA), according to the manufacturer's instructions. To construct a normalized full-length cDNA library, the SMART cDNA library construction kit (Clontech, USA) and TRIMMER-cDNA normalization kit (Evrogen, Russia) were used according to the manufacturer's instructions. The cDNAs were fractionated using CHROMA SPIN-400 column (Clontech). All cDNAs larger than 500 bp were modified with two different *Sfi*I restriction enzyme sites for insertion in the MCS and ligated into pcDNA3,1(+) vector (Invitrogen), then transformed into TOP10 *Escherichia coli* (Invitrogen).

### 1-5. Screening of putative p53-interacting proteins

Pools of human cDNAs from HeLa and liver Chang cells were transcribed and translated *in vitro* using the TnT Coupled Reticulocyte Lysate System (Promega, USA) in the presence of ³⁵S-methionine (Perkin Elmer, USA). When a protein pool was confirmed positive in the *in vitro* binding assay, the corresponding cDNA pool was subdivided and re-examined in the same manner until a single positive cDNA clone was isolated. Positive clones were sequenced (Bionics, Korea) and compared with known sequences by searching the NCBI gene databases.

### 1-6. Purification of GST-fusion proteins

Expression of GST fused recombinant p53, Hades (WT, FL, RP, and RP MT) and Mdm2 proteins in BL21 cells was induced by 0.1 mM IPTG at 37°C for 2 h or at 25°C overnight. Proteins were lysed by sonication in bacterial lysis buffer (30 mM Tris-HCl pH 8, 100 mM NaCl, 0.1 mM EDTA, 1 mM DTT, and 1% NP40) and purified using glutathione-Sepharose (Amersham Bioscience). The expression of His-tagging p53 and p53(K24R) mutant were induced in BL21 cells by incubating with 0.1 mM IPTG at 37°C for 4 hr and purified using Ni-NTA beads.

### 1-7. In vitro binding assay

³⁵S-labeled proteins obtained by *in vitro* transcription/translation were incubated with 5 µg glutathione-Sepharose 4B bound GST, GST-p53, or GST-Hades recombinant proteins for 3 h at 37°C in binding buffer (30 mM Tris-HCl pH 8.0, 0.1 mM EDTA, 0.1 mM NaCl, 1 mM DTT, 1% NP-40, and 0.5 mM PMSF), then washed five times and boiled in SDS-sample buffer (60 mM Tris-Cl pH6.8, 25% glycerol, 2% SDS, 14 mM 2-mercaptoethanol, 0.1% bromophenol blue). The bound proteins were separated by SDS-PAGE and visualized by autoradiography.

### 1-8. Ubiquitination assay

For *in vitro* p53 ubiquitination assays, 1 µl of *in vitro* translated ³⁵S-labeled protein was incubated in 20 µl ubiquitination buffer (25 mM Tris-HCl pH 7.5, 1 mM DTT, 2 mM ATP, 0.06 % NP40, 5 mM MgCl₂, and 15 µM ZnCl₂) in the presence of 1 µg indicated E3 ligase, 150 ng purified E1, 150 ng E2 enzymes, and 10 µg ubiquitin. After incubation at 30 °C for 2 h, reaction products were analyzed by SDS-PAGE followed by autoradiography or immunoblotting.

For endogenous p53 ubiquitination assay, MCF7 cells were harvested and resuspended with ubiquitination buffer. The sample was centrifuged after briefly sonication. Equal amount of the supernantants were incubated with GST, GST-Hades, or GST-Hades MT for 2 h at 30°C followed by immunoprecipiation using anti-p53 or anti-ubiquitin antibody. The immunoprecipitates were by immunoblotting.

For p53 ubiquitination assay in MEF Mdm2^{-/-}, p53^{-/-} cells, lysates were prepared after transfected with pcDNA myc/His1-p53 expression plasmid for 48 hr. The reactions were performed as the same above. The immunoprecipitates were purified with anti-p53 antibody or pull-down with Ni⁺ conjugated beads followed by immunoblotting.

For *in vivo* p53 ubiquitination assay, HCT116 p53^{-/-} or H1299 cells were transfected with GFP, GFP-Hades, or GFP-Hades RING MT. At 48 hr after transfection, cells were treated with 10 µM MG132 for 4 hr. The ubiquitinated lysates were analyzed by immunoprecipitation using anti-p53 antibody followed by immunoblotting using anti-Ubiquitin antibody. *In vivo* p53 ubiquitination analysis, HCT116 p53^{-/-} or H1299 cells were transformed with GFP, GFP-Hades or GFP-Hades RING MT for 48 hr. After the transformation, cells were incubated for 4 hr using 10 µM MG132. Then, cells were lysed using SDS-containing lysis buffer and heated for 10 hr. Cells were diluted ten times using NP40 lysis buffer for anti-p53 antibody immunoprecipitation. The anti-p53 immunoprecipitate was analyzed by immunoblotting using anti-polyubiquitin antibody.

### 1-9. Immunoprecipitation and immunoblotting

Cells were lysed with SDS-containing buffer (20 mM Tris-Cl pH 7.4, 1 % SDS, and 2 mM EDTA) for 5 min on ice. Lysates were diluted 10-fold with resuspension buffer (20 mM Tris-Cl pH 7.4 NP40 0.5%, 150 mM NaCl, and 2 mM EDTA), centrifuged at 1,200 rpm for 30 min. The supernatants were incubated with antibody overnight at 4°C. Following incubation with protein A or protein G conjugated agarose for 2 h, the beads were washed six times with resuspension buffer, then SDS-sample buffer was added and boiled for 10 min. Immunoblotting analysis was performed as previous described [Brooks et al., Molecular Cell 21, 307-315 (2006)].

### 1-10. Quantitative real-time PCR

To measure the *Hades* mRNA level in human HCC, we obtained 28 HCC and adjacent liver tissues and normal liver tissues from patients at the Seoul National University School of Medicine with the approval of the Ethics Committee (Seoul, Korea). RNAs were isolated from tissues using RNeasy mini kit (Qiagen, Germany), according to the manufacturer's instructions. The expression value of *Hades* mRNA was quantified by real-time PCR using IQ SYBR Green Supermix (Bio-Rad, USA) and CFX96 real time system (Bio-Rad). The equal mixture of RNA extracted from five differnet normal livers without liver cirrhosis and fibrosis was also included in real-time PCR, and *18S* rRNA was used as a reference control in both RT-PCR and real-time PCR analysis. Briefly, *Hades* mRNA was amplified with primers (forward GATCATTCATCAGAGGACCAACACAG; reverse AGCACTCGCACAGCCACATC). *18S* rRNA sequence was amplified with primers (forward GGAGAGGGAGCCTGAGAAACG; reverse TTACAGGGCCTCGAAAGAGTTC). Expression value of *Hades* mRNA was analyzed with the CFX Manager Software (Bio-Rad).

### 1-11. Immunofluorescence assay

Transfected cells grown on glass coverslips were fixed with 4% paraformaldehyde for 5 min at room temperature, washed twice with PBS, and then incubated in permeabilization buffer (0.5% Triton X-100 in PBS) for 3 min. Cells were blocked with 5% BSA with 2% of Goat serum in PBS for 30 min, then incubated with anti-p53 antibody (1:200 in PBS) overnight at 4°C, washed three times with PBS, and further incubated with fluorescein Texas red-labeled secondary antibodies (1:500 in PBS) for 1 h. Coverslips were inverted, mounted on slides with Vectashield (Vector Laboratories, USA), and fixed with nail polish. Fluorescence was monitored using an confocal lazer scanning microscope (FV-1000 spectral, Olympus)

### 1-12. Subcellular fractionation

Cells were harvested, rinsed with PBS, and pelleted. The cells were resuspended in CLB buffer (10 mM HEPES, 5 mM NaHCO₃, 10 mM NaCl, 1 mM CaCl₂, 1 mM KH₂PO₄, 0.5 mM MgCl₂, and 5 mM EDTA) and allowed to swell on ice for 5 min, then NP40 was added to a final concentration of 0.5% and incubated for 2 min on ice. The lysate was centrifuged at 1,300 rpm for 5 min and the supernatant was transferred to a fresh tube for further centrifugation at 12,000 rpm for 30 min. The supernatant from this tube was collected as the cytoplasmic fraction. The crude nuclear pellet was resuspended in 1 ml of CLB buffer and washed three times. Nuclear and cytoplasmic fractions were added to SDS sample buffer. Mitochondrial fractionation was performed as previous described [Shirangi et al., FASEB J. 16, 420-422 (2002)]. The mitochondrial fraction (pellet) was washed with corresponding buffer for three times and added to SDS-sample buffer, boiled for 10 min at 100°C, and analyzed by immunoblotting as indicated.

### 1-13. Cell viability assays

For the MTS assay, cells were incubated with CellTiter 96® AQueous Non-Radioactive Cell Proliferation Assay reagent (Promega, USA) for 1 h, and then the O.D. was measured at 490 nm. Apoptosis, DNA content, and mitochondrial membrane potential were determined by flow cytometry using annexin V-PE, propidium iodine, and JC-1 staining, as previous described [Shirangi et al., FASEB J. 16, 420-422 (2002)].

### 1-14. Colony formation assay

Totals of 5x10³ or 1x10⁴ HCT 116 p53^{+/+} or HCT 116 p53^{-/-}, H1299 or p53^{-/-};Mdm2^{-/-} cells were plated in a 60-mm dish and transfected with 500 ng of the indicated plasmid for 24 h. Cells were placed under G418 selection (500 µg/ml) for 14 days, fixed, and stained with crystal violet for colony counting.

### 1-15. Luciferase assay

Aliquots of 5x10⁴ cells were plated in 24-well plates. The following was added to each well: 500 ng of pGL plasmid containing the promoter p53 binding site for *Bax* or *Puma,* 100 ng of p53 plasmid, and 200 ng of Hades or Hades MT plasmid. At 48 h after transfection, cells were harvested and cell extracts were prepared by addition of 50 µl Passive lysis buffer (Promega). Luciferase activities were measured using a Biotek synergy HT microplate reader. The relative luciferase activities were normalized by mesuring β-galactosidase activities using Luminescent β-galactosidase detection kit II (Takara-Clontech).

### 1-16. Statistical analysis

Statistical analysis was performed using the two-tailed Student's t test. Statistical significance was determined as P < 0.05.

### EXAMPLE 2. Result

### 2-1. Novel p53-binding protein

To identify proteins that bind p53, we prepared a human full-length cDNA library derived from HeLa and liver Chang cells. The *in vitro* transcribed and translated protein pool from cDNA library was used to screen the p53 binding proteins using modified SMART technology (Fig. 1a). An average of more than 100 different proteins was contained in the ³⁵S-labeled protein pool. We systematically validated more than 9,600 different ³⁵S-labeled proteins *in vitro* and isolated cDNA pool positive for interaction with GST-p53. After identifying the potential p53 interacting proteins in the protein pool (for example, pool #D5 in Fig. 1b), the corresponding cDNA pool was progressively subdivided and re-examined in the same manner until a single positive cDNA clone was isolated. Among the isolates, one clone was identified for a major p53-binding partner. This protein, which we have named Hades, contains several predicted functional domains (Fig. 1c): a transmembrane (TM) domain or signal peptide in the N terminus a second TM domain in the middle, and a RING-finger domain (i.e., the signature E3 ligase domain) in the C terminus. Confocal microscopic images showing Hades localization in the mitochondria of U2OS cells, which were cotransfecfied with mitotracker and control GFP (upper panel) or GFP-Hades (lower panel) plasmid. The confocal microscopy revealed that Hades was localised distinctly in mitochondria (Fig. 1d).

Next, we verified that Hades is a *bona fide* p53-interacting protein *in vitro* and *in vivo. In vitro* translated, ³⁵S-labelled p53 interacted with GST-Hades (left panel). *In vitro* translated, ³⁵S-labelled Hades interacted with GST-p53 (right panel). The pull-down assays revealed that GST-p53 interacted with *in vitro* translated Hades and GST-Hades interacted with *in vitro* translated p53 (Fig. 1e). To identify the binding site between p53 and Hades, an *in vitro* translated, ³⁵S-labelled N-terminal (aa 1-988) or C-terminal (aa 187-393) fragment of p53 was incubated with GST-Hades, and bound proteins were detected by autoradiography. Consequently, GST-N-p53 (aa 1-186) associated with immobilized GST-Hades, whereas GST-C-p53 (aa 187-393) did not (Fig. 1f). Furthermore, MCF7 cell lysates were incubated with GST (lane 2) or GST-Hades (lane 3). The level of p53 was assessed by immunoblotting using anti-p53 antibody and by *in vitro* GST pull down assay. The result demonstrated that Hades interacted with p53 in MCF7 cell (Fig. 1g). We identified the *in vivo* interaction between Hades and p53. p53 null H1299 cells were cotransfected with HA-p53 and GFP-Hades expression plasmids. At 24 h after transfection, the immunoprecipitates by anti-GFP antobody and immunoblotting using anti-p53 antibody were performed. The *in vivo* interaction between Hades and p53 was detected by immunoprecipitation of GFP-Hades binding complex (Fig. 1h). Moreover, The interaction between p53 and Hades in MCF10A and NHLF cells were evaluated in *vivo* by coimmunoprecipitation. p53 and Hades were immunoprecipitated from normal breast MCF10A and lung fibroblast NHLF cell lysates with anti-p53 antibody or anti-Hades antibody at 12 h after MG132 treatment. The levels of Hades and p53 were then analyzed by immunoblotting. The interaction between Hades and p53 was observed in both MCF10A and NHLF cells (Fig. 1i). U2OS cells were transfected with GFP-Hades expression plasmids and 24 h later, incubated under normal condition (upper panel) or in the presence (lower panel) of MG132 for 4 h. Cells were then fixed, successively probed with p53 antibody and Texas Red-conjugated secondary antibody, and visualized by confocal microscopy. Endogenous p53 was mainly observed in the nucleus of MG132-treated, Hades-nontransfected U2OS cells. However, the amount of p53 was localized out of nucleus, as shown in a merged image (Fig. 1j). Quantitation of these results demonstrated that more than 30% of cells were analyzed the colocalization of p53 and Hades (Fig. 1j bottom). These results indicate that Hades and p53 are colocalized in the mitochondria. Collectively, these data reveal that Hades and p53 interact both *in vitro* and *in vivo.*

### 2-2. Reduction of Level of p53 though RING ligase activity

To investigate the possible regulation of p53 by Hades, we determined the effect of Hades on endogenous U2OS cells were transfected with increasing amounts of GFP-Hades plasmid. After 24 h further incubation the levels of ectopically expressed Hades and endogenous p53 were examined by immunoblotting with anti-GFP and anti-p53 antibodies. As shown in Figure 2a, ectopically expressed Hades reduced the p53 level in a dose-dependent manner. The ectopically expressed Hades reduced the level of p53 in a dose-dependent manner. This decrease in p53 was also observed in immortalized breast epithelial cells (MCF10A) and normal human lung fibroblast (NHLF) cells transfected with GFP, GFP-tagged wild-type Hades, or GFP-tagged mutant Hades (Fig 2b).

To investigate whether the E3 ligase activity of Hades is involved in mediating this reduction in p53, the autoubiquitination activities of Hades, Hades RING finger peptide (RP), mutated Hades, and Mdm2 were investigated. GST, GST-Hades, GST-Hades RING peptide, GST-Hades RING-mutant peptide (C302S/C305S) [Wu et al., Nat Genet. 14, 430-440 (1996)], and GST-Mdm2 were incubated with or without E1/E2 (UbcH5c) at 30°C for 2 h in ubiquitination buffer. Bound protein on glutathione-beads were washed six times with ubiquitination buffer followed by immunoblotting using an anti-ubiquitin antibody. The result indicated that all of them have ubiquitination activities in present of E1/E2 except for RING-mutant Hades (Fig. 2c). Moreover, H1299 cells were transfected with plasmids expressing p53, GFP-tagged Hades, or FLAG-tagged mutant Hades. At 24 h after transfection, cells were harvested and p53 levels were assessed by immunoblotting using anti-p53 antibody. RING mutation Hades failed to reduce the p53 level (Fig. 2d). Thus, RING mutation Hades did not induce Hades-mediated p53 degradation, wild type(WT) Hades serve a role in downregulated p53 function.

To gain a more detailed insight into the function of Hades in p53 downregulation, we investigated the regulation of p53 stability by Hades. H1209 cells were transfected with each indicated plasmid for 24 h and untreated or treated with 90 µM MG132 for 4 h. The reduced level of p53 by Hades was recovered by treatment of MG132 (Fig. 2e). To generate of stable Hades-knockdown MCF7 cells using the shRNA, the cells were transfected with pSuper-Hades 1 or 2. After selection for 14 days in media containing G418 (500 µg/ml), *hades* mRNA levels were measured by RT-PCR and the levels of endogenous Hades and p53 proteins were measured by immunoblotting. Consequently, we confirmed that p53 was stabilized by silencing of Hades (Fig. 2f). Furthermore, Stable Hades-knockdown MCF7 cells using Hades shRNA were incubated with cycloheximide (CHX, 150 µg/ml). The half-life of p53 protein was greater in pSuper-Hades transfected cells (lower panel) than in control (upper panel) (Fig. 2g).

Next, we monitored the localization of p53 after the knockdown of Hades. The p53 level of stable Hades-knockdown MCF7 cells was greater than that of control (upper panel). The levels of endogenous p53 were measured in fractionated extracts of stable MCF7 cells. We confirmed that p53 in the both nucleus and cytoplasm was accumulate (Fig. 2h). Since p53 and Hades colocalized outside of the nucleus (Fig. 1j), we further examined whether cytoplasmic localization of p53 is important for the reduction of p53 level by Hades. H1299 cells were transfected with each plasmid expressing p53 and GFP, GFP-Hades or GFP-Hades mutation. Treatment with the Crm1 inhibitor leptomycin B (LMB) restored the level of p53 (lane 2-lane5) (Fig. 2i). These indicate that Hades-madiated nuclear export of p53 was blocked and level of p53 was restored by LMB treatment. And also, we used a p53 expression plasmid encoding a mutant p53 (C153Y) that is localized mainly to the cytoplasm but not in the nucleus [Nie et al., J Biol Chem. 282, 14616-14625 (2007)]. We performed immunoblotting assay of ectopically expressed wild-type or mutant p53(C135Y) . H1299 cells cotransfected with 0.5 µg wild-type or mutant (C135Y) p53 plasmid and GFP-Hades plasmid (0, 0.2, 0.5, 1.0, 2.0 or 4.0 µg). At 24 h after transfection, ectopically expressed wild-type p53 and mutant p53 levels were measured by immunoblotting. In case of transfection with GFP-Hades plasmid of same dose, ectopically expressed Hades reduced p53 more effectively in C153Y p53 mutant expressing H1299 than in wild-type p53 expressing H1299 cells (Fig. 2j).

These data indicate that Hades downregulates the cytoplasmic p53 by ubiquitin-dependent degradation pathway.

### 2-3. Promotion of polyubiquitination of p53 by Hades

To investigate whether Hades is directly responsible for p53 ubiquitination, we performed autoubiquitination assay as describe 2-2. The assay showed that both immobilized full-length (lane 2, GST-Hades) and a RING-finger domain (aa 271-351) [lane 4, GST-Hades Ring protein (RP)] exhibited autoubiquitination, whereas the RING mutant peptide (lane 6 C302S/C305S; GST-Hades RP MT) did not (Fig. 2c).

Subsequently we performed *in vitro* ubiquitirtation assay after Hades interacts with p53 protein. *In vitro* translated ³⁵S-labeled wild-type or mutant p53 (wild-type: lanes 1-3; 5NKR: lanes 4-6; 6NKR: lanes 7-9; and K24R: lanes 10-12) was incubated with GST or GST-Hades. The interaction between GST-Hades and p53 protein was analyzed by GST pull-down assays (Fig. 3a).

Since there are several E2 conjugating enzymes which participate in the ubiquitin-mediated degradation [Smalle et al., Annu Rev Plant Biol. 2004, 555-590 (2004)], we examined which E2 enzyme is responsible for the ubiquitination of p53 by Hades. *In vitro* translated ³⁵S-labeled p53 was incubated with GST-Hades RP in the presence of one of ATP, His-tagged ubiquitin E1 and several E2 enzymes (Fig. 3a). After *in vitro* ubiquitination, samples were examined SDS-PAGE and detected using autoradiography. The ³⁵S-labeled p53 was polyubiquitinated in an Ubc5a or Ubc5c-dependent manner (lane 5 and 7, Fig 3b). Moreover, *in vitro* translated ³⁵S-labeled p53 was incubated with GST, GST-Hades RP or GST-Mdm2 in the presence of ATP, His-tagged ubiquitin E1 and E2(UbcH5c). p53 ubiquitination was assessed by immunoblotting using anti-p53 antibody. We confirmed that p53 ubiquitination by Hades was usually responsible for general ubiquitin-mediated degradation as well as Mdm2-mediated p53 ubiquitination (Fig. 3c). To confirm a direct role for Hades in p53 ubiquitination, we performed *in vitro* ubiquitination assay. *In vitro* translated ³⁵S-labeled p53 was incubated with GST, GST-Hades RP, or GST-Hades RP mutation in a reaction containing ATP and His-ubiquitin, E1, and E2 (UbcH5c). The p53 ubiquitination was assessed by immunoblotting using anti-p53 antibody. The p53 ubiquitination was only induce by Hades (Fig. 3d). To confirm a direct role for Hades in p53 ubiquitination, we performed *in vitro* ubiquitination assay of E. *coli*-drived recombinant p53 (Fig. 3d). This experiment verified the involvement of Hades in p53 ubiquitination *in vitro* in the presence of E1, E2 (UbcH5c) and ubiquitin. For *in vitro* ubiquitination assay, p53 null cells H1299 cells were cotransfected with expression plasmids for p53 (2 µg) and GFP-Hades, or GFP-Hades MT (4 µg). At 24 h after transfection, cells were further incubated under normal condition or in the presence of MG132 (10 µM) for 4 h. After the incubation, cells were harvested and lysed. The whole cell extracts were immunoprecipitated using anti-p53 antibody. The ubiquitin-conjugated p53 proteins were examined by immunoblotting using anti-polyubiquitin antibody. The polyubiquitinated p53 was only detected in Hades-transfected cells treated with MG132 (Fig. 3e).

We tested whether the mechanism of p53 ubiquitination by Hades differed from that of ubiquitination by Mdm2. Plasmids expressing p53(1 µg) and GFP-Hades or GFP-Hades MT(1 µg) were transfected in MEF cells lacking both p53 and Mdm2 (MEF *p53*^{*-*/}*⁻ mdm2*^{*-*/*-*} cells). We found reduced p53 level in the presence of GFP-Hades in MEF *p53*^{*-*/}*⁻ mdm2*^{*-*/*-*} cells (Fig. 3f, lane 2). Moreover, MEF *p53*^{*-*/}*⁻ mdm2*^{*-*/*-*} cells were transfected with plasmid for His-p53. At 24 h after transfection, cell lysates were harvested and *in vitro* ubiquitination assay of the lysates containing GST, GST-Hades RP, or GST-Hades RP MT were performed for 2 h. Then, p53 was pulled down with Ni⁺-conjugated beads (left panel) or immunoprecipitated using anti-p53 antibody (right panel). *In vitro* ubiquitination assays demonstrated polyubiquitinated p53 in the presence of GST-Hades RP but not GST-Hades RT MT in MEF *p53*^{*-*/}*⁻ mdm2*^{*-*/*-*} cells (Fig. 3g). Altogether, MEF p53^{-/-} mdm2^{-/-} cells were cotransfected with plasmids expressing p53, GFP- Hades, and Mdm2 as indicated. At 24 h after transfection, in the cells co-transfected with GFP-Hades and Mdm2 plasmids, p53 ubiquitination effects were great than in the cells transfected only with GFP-Hades (Fig. 3h). These data reveal that Mdm2 is dispensable for ubiquitination of p53 by Hades.

In accordance with previous reports, specific six lysine residues in the C-terminus of p53 are mainly ubiquitinated sites by Mdm2[Rodriguez et al., Mol Cell Biol. 20, 8458-8467 (2000)]. Therefore, we monitored which is the critical lysine residue for the Hades-mediated ubiquitination of p53. As shown in Fig. 3i, we prepared p53 lysine mutation: 5NKR, mutated at five N-terminal lysine residues (aa 101, 120, 132, 139, 164); 6NKR, mutated at six N-terminal lysine residues (aa 24, 101, 120, 132, 139, 164); 6CKR, mutated at six C-terminal lysine residues (aa 370, 372, 373, 381, 382, 386); and N24KR, mutated at one N-terminal lysine residue (aa 24). Then, *In vitro* transfected p53(WT, 5NKR, 6NKR or 6CKR) was incubated with GST or GST-Hades RP in ubiquitin buffer, the p53 levels were measured by immunoblotting. The data suggest that none of these lysine residues are necessary for this process as Hades was still able to ubiquitinate a form of p53 in which all six lysines was mutated (6CKR) (Fig. 3j). Instead, a comparison of p53 mutants with substitutions at five or six of the N-terminal lysines (5NKR or 6NKR) showed that lysine 24 is a critical residue for stabilization of p53 by Hades (Fig. 3j). To examine effects of lysine 24 on Hades-induced p53 ubiquitination, *in vitro* translated, ³⁵S-labelled WT p53 or mutant p53 in which lysine 24 residue is substituted with arginine (mutant p53 K24R) was incubated with GST, GST-Hades RP or GST-Hades RP MT in ubiquitination buffer. Reaction products were analysed by SDS-PAGE and autoradiography. As expected, WT p53 was ubiquitinated by Hades, but the *in vitro* translated p53 K24R was not ubiquitinated by Hades (Fig. 3k (A)). GST pull-down assays showed that a defect in ubiquitination of the p53 K24R mutant was not due to a failure to interact with Hades (Fig. 3k (B)).

These results support that the Hades-mediated polyubiquitination of p53 is dispensable for Mdm2 and implicate that the lysine 24 residue of p53 is critical for the ubiquitin-dependent degradation of p53.

### 2-4. Effect of Hades on p53-dependent growth suppression and apoptosis

Since E3 ligase activities necessary for p53 ubiquitination can also regulate p53 function [Dornan et al., Nature 429, 86-92 (2004); Leng et al., Cell 112, 779-791 (2003)], we explored the effect of Hades on p53-dependent growth suppression and apoptosis. First, we examined whether Hades affects p53-mediated transcriptional activation using a luciferase reporter assay (Fig. 4a). H1299 cells were cotransfected with expression plasmids for p53 and GFP, GFP-Hades, or GFP-Hades MT, together with Bax-luc or Puma-luc reporter plasmid. At 24 h after transfection, the relative luciferase activities were measured by luminometer. As shown in Figure 4a, activation of Bax-1 and PUMA-luc was decrease by head, this p53-mediated transactivation was suppressed by Hades. However, H1299 cells were cotransfected with expression plasmids for p53 and GFP, GFP-Hades, or GFP-Hades MT, together with Bax-luc or Puma-luc reporter plasmid. At 24 h after transfection, cells were treated with LMB (20 nM) for 4 h before the relative luciferase activity was measured. p53 transcriptional activity was not reduced by Hades in the presence of LMB, the expressions of endogenous p53 downstream genes Bax and Puma weren't downregulated by mutant Hades(Fig. 4b). However, H1299 cells were cotransfected with expression plasmids for p53 and GFP, GFP-Hades, or GFP-Hades MT, together with Bax-luc or Puma-luc reporter plasmid. At 24 h after transfection, luciperase activation and p53 experssion were measured. As a result, it was analyzed that the degradation of mutant p53 depleted with transactivation activity was mediated by Hades, demonstrating that Hades-mediated degradation of p53 was independent of its transactivation ability (Fig. 4c).

Because p53 accumulation leads cell growth suppression [Finlay et al., Cell 57, 1083-1093 (1989)], we investigated whether Hades regulates the p53-dependent growth suppression using MTS assay. MTS cell proliferation assays were performed on HeLa, MEF p53-/- mdm2-/-, MCF7, and H1299 cells at 72 h after transfection with 500 ng of plasmid encoding p53 and GFP, GFP-Hades, or GFP-Hades MT(Fig. 4d). H1299 cells were cotransfected with plasmids expressing p53 along with GFP or GFP-Hades and then incubated for 14 days in media containing G418 (500 µg/ml). Cell growth was visualized by crystal violet staining. We confirmed that Hades inhibits p53-mediated growth suppression using colony-formation assays in H1299 cells (Fig. 4e).

Next, using RNA interference system, we investigated whether Hades inhibits p53-dependent growth suppression. HCT116 p53+/+ and HCC116 p53-/- cells were evaluated by colony formation assay. Cells were cotransfected with 1 µg pSuper control plasmid or pSuper-Hades plasmid. Cells were further incubated with media containing G418 (500 µg/ml) for 14 days. Hades suppression restored p53-dependent growth suppression in HCT116 p53+/+ cells but not in HCT116 p53-/- cells(Fig. 4f). And, Hades-knockdown reduces the cell viability under various stress conditions. Stable Hades-knockdown MCF7 cells (gray bar) and control MCF7 (black bar) cells were incubated with treating H₂O₂ (10 µM), actinomycin D (1 µM), or doxorubicin (500 nM) for 24 h. The cell viabilities were measured using MTS assay. Hades knockdown restored the p53-mediated growth suppression in Hades-knockdown cell(Fig.4g).

Thus supporting p53 acts as a central switch to induce growth suppression in response to a variety of cellular signals[Dulic et al., Cell 76, 1013-1023 (1994); Lowe et al., Nature 362, 847-849 (1993); Lane DP, Nature 358, 15-16 (1992)], these data suggest that Hades negatively regulates the p53-mediated growth suppression. Thus, Hades appears to act as a negative regulator of p53, protecting cells from apoptosis under various stress conditions.

### 2-5. Regulation of exonuclear function of p53 by Hades

In recent reports, p53 has been known to distribute throughout the nucleus and cytoplasm under normal conditions[Shaulsky et al., Oncogene 5, 1707-1711 (1990); Martinez et al., Genes Dev. 5, 151-159 (1991)]. Moreover, Our results showed that Hades is colocalized with p53 in the exonuclear (Fig.1j). This, taken with the fact that cytoplasmic and mitochondrial p53 triggers stress-apoptosis by interacting with the bcl-2 family[Green et al., Nature 458, 1127-1130 (2009)], suggests that Hades interacts with p53 under certain conditions to control p53 function.

Therefore, we investigated whether Hades regulates mitochondrial p53 by using U2OS cells transfected with pEYFP(enhanced yellow fluorescent protein)-mito-p53, which encodes p53 coupled to the mitochondria-targeting signal. After transformation of pEYFP-mito-p53 into U2SO cells for 24 hr, cells were observed under a confocal microscope. As a result, pEYFP-mito-p53 was observed to be localized in mitochondria. Then, to verify the interaction between p53 and Hades, HEK293 cells were cotransformed with EYFP-mito-p53 and pCI-BcI-2 expressing plasmids in the presence or absence of FLAG-Hades and FLAG-Hades MT and lysed. The cell lysis was immunoprecipitated using BcI-2 antibody. Interestingly, ectopically expressed Hades reduced the interaction between pEYFP-mito-p53 and BcI-2 although the level of p53 is abundant enough as control cells in the presence of MG132 (Fig. 5b, lane 3). Moreover, the expression of the RING-finger domain deleted mutant Hades did not affect the interaction between mitochondrial p53 and BcI-2 (Fig. 5b, lane 4).

We also tested the effect of Hades on cell proliferation and found that colony formation. H1299 cells were cotransfected with plasmids expressing YFP-tagged mitochondria-localizing p53 (pEYFP-Mito-p53) along with GFP or GFP-Hades and then stained with crystal violet after incubation for 14 days in media containing G418 (500 µg/ml). As a result, colony formation was decreased (Fig. 5c). Also, MEF p53-/-mdm2-/- cells were cotransfected with plasmids expressing EYFP-mito-p53 or EYFP-mito-p53 (K24R) along with GFP or GFP-Hades. At 14 days after transfection, cell growth was visualized by crystal violet stain. It was shown that the cell growth inhition by Hades was not suppressed in cells expressing EYFP-mito-p53 mutants (Fig. 5d).

In a prior research, as p53 is rapidly translocated to mitochondria upon camptothecin (CPT) treatment for its exonuclear function [Mihara et al., Molecular Cell 11, 577-590 (2003)]. We examined whether Hades would be able to weaken the exonuclear function of p53 upon CPT treatment. MCF7 cells were transfected with plasmids (1 µg) expressing GFP or GFP-Hades. At 24 h post-transfection, cells were treated with CPT (1 µM) for 6h. Accumulation of p53 upon CPT treatment was attenuated by Hades expression(Fig. 5e, upper panel). MCF7 cells were transfected with expression plasmid for GFP or GFP-Hades RING MR. At 24 h after transfection, cells were treated with CPT [5 µM] for 6 h and their nuclear and cytoplasmic fractions were collected to measure the expression level of p53. It was shown that the expression of p53 was accumulated in nucleus and cytoplasm by CPT treatment and its cytoplasmic expression was decreased by Hades (Fig. 5e, lower panel). Further, MCF7 cells were transfected with the expression plasmids for GFP or GFP-Hades MR. At 24h after transfection, cells were treated with 1 µM CPT for 24 h. Cells were harvested, lysed and immunoprecipitated with anti-p53 antibody. As a result of measuring the expression level of BcI-2 and p53 using immunoblotting, accumulation of p53 expression was observed with treatment of CPT. Hades inhibits the interaction between p53 and BcI-2 in CPT-treated cells.(Fig. 5f) In order to confirm the effect of Hades on CPT-induced apoptosis, MCF7 cells were transfected with plasmids expressing GFP or GFP-Hades. At 24 h post-transfection, cells were treated with CPT (1 µM) for 24 h. Then cell death number was observed with Flow cytometry. As a result, it was demonstrated that Hades inhibits CPT-induced apoptosis (Fig. 5g).

Next, the above effects were confirmed using RNA interfereing system. MCF7 cells were transfected with pSuper or pSuper-Hades, and the cells were treated with 1 µM CPT for 24 h. Cells were harvested, lysed and immunoprecipitated using anti-p53 antibody. The immunoprecipitates were analyzed by immunoblotting using anti-BcI-2 and anti-p53 antibodies. As a result, the expression of p53-BcI-2 complex was increased in stable Hades knock-down MCF7 cells following treatment of CPT more than in control cells (Fig. 5h, lane 3 vs 4), and the expression of p53 was also considerably increased. Cytochrome C release from the mitochondrial fraction in Hades knock-down MCF7 cells following treatment of 1 µM CPT was measured by immunoblotting using an anti-cytochrome C antibody. As a result, it was shown that CPT treatment induced release of cytochrome C from mitochondria (Fig. 5h, lower panel).

The present inventors investigated the negative role of Hades on p53-dependent apoptosis additionaly using FACS analysis. MCF7 cells were transfected with pSuper or pSuper-Hades, treated with 1 µM CPT for 24 h, and analyzed by FACS to measure the sub-G0 fraction. As a result, CPT treatment was higher in Hades knockdown MCF7 cells than in control cells(Fig.5i). Further, HCT116 p53+/+ and HCT116 p53-/- cells were transfected with 40 nM Hades siRNA or control siRNA. After incubation for 24 h, cells were further treated with 1 µM CPT for 24 h. Cells were harvested and stained with JC-1 for 30 min. As a result, the loss of mitochondrial membrane potential was induced after CPT treatment was only detectable in HCT116 p53+/+ cells but not in HCT116 p53-/- cells (Fig. 5j). These results suggest that Hades interacts rapidly with p53 and inhibits its exonuclear function by preventing the interaction between Bcl-2 and p53.

In a prior research, the exonuclear function of p53 serves a tumor suppressor role[Talos et al., Cancer Res. 65, 9971-9981 (2005)], and other ligases that ubiquitinate p53 are highly expressed in some tumor tissues[Dornan et al., Cancer Res. 64, 7226-7230 (2004); Wenrui et al., Journal of the National Cancer Institute 96, 1718-1721 (2004)]. The present inventors determined the expression level of Hades in human tumor tissue. The level of *Hades* mRNA was evaluated in 28 pair-matched liver biopsies from human hepatocellular carcinoma (HCC) and from adjacent normal tissues. Interestingly, 2 fold of higher mRNA level of *Hades* was observed compared with adherent normal tissue (Fig. 5k). These results were consistent with the hypothesis that increased Hades expression affects liver tumorigenesis by reducing p53 activity. Taken together, our data show that Hades inhibits the exonuclear tumor suppressor function of p53 through ubiquitination and subsequent proteasomal degradation of p53.

Here, the present inventors have identified Hades as a p53-interacting protein that acts as an E3 ligase for exonuclear p53 and degrades it through the ubiquitin-dependent pathway. Previous reports has been identified that Hades is a RING-finger domain localized mainly to mitochondria (Fig. 1d) and Sumoylates dynamin-related protein 1 (Drp-1) result in regulation mitochondrial dynamics[Li et al., PLOS ONE 3, e1487 (2008); Braschi et al., EMBO Rep. 10, 748-754 (2009)]. Recently, Hades-induced cellular cytotoxicity through JNK pathway has also reported[Zhang et al., Cell Res. 18, 900-1000 (2008)]. However, we newly found that Hades has a function on modulating p53. Immunofluorescence analysis shows that Hades interacts with p53 out of nucleus (Fig. 1j) and ectopic expression of Hades causes degradation of cytoplasmic p53 (Fig. 5e, lower panel). Based on our observations, we proposed that ubiquitination of p53 by Hades in the cytoplasm is responsible for reducing p53 levels in both cytoplasmic and mitochondrial compartments.

Although there are several E3 ligases including Mdm2 targeting p53 have been reported[Dornan et al., Nature 429, 86-92 (2004); Leng et al., Cell 112, 779-791 (2003)], we suggest that Hades has a distinct mechanism against Mdm2 for those reasons. First, where as mdm2 reduces p53 levels in the nucleus 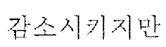 [Shirangi et al., FASEB J. 16, 420-422 (2002)], Hades reduced the level of p53 in the cytoplasm (Fig. 5e lower panel) and ubiquitinated p53 by Mdm2-independent manner (Fig. 3f). Second, the N-terminai lysine residue of p53 (lysine 24) which is not reported to be ubiquitinated by Mdm2, is critical for ubiquitination by Hades (Fig. 3k.(A)). Third, Hades did not have negative feecback loop. UV irradiation or ectopic expression of p53 does not induce an increase in Hades mRNA in A549 or HCT116 cells (data not shown). Several candidate cytoplasmic E3 ligases targeting p53 have been reported as E3 ligase in normal status, including ARF-bp1 and p300[Chen et al., Cell 121, 1071-1083 (2005); Shi et al., Proc Natl Acad Sci U S A. 106, 16275-16280 (2009)]. However, we cannot rule out the possibility that the other E3 ligase pathway may also be contributing to Hades-mediated p53 modulation.

Interestingly, we showed that knockdown of Hades leads accumulation of p53 both in nucleus and cytoplasm (Fig. 2h). It is well known that the protein stability and transactivation of p53 are tightly regulated by a variety of biochemical mechanisms. According to recent reports, ubiquitinated cytoplasmic p53 regulated its stabilization as well as localization. The ubiquitinated form of p53 was unable to interact importin for its nuclear import [Marchenko et al., Cell Death Differ. 17, 255-267 (2010)], and deubiquitination of p53 by Usp 10 result in p53 nuclear import and activation. Therefore, it is likely that p53 accumulates both in nucleus and cytoplasm by ablation of Hades (Fig. 2h)

Our data highlight that Hades regulates exonuclear function of p53. Hades inhibited tumor suppressor function of mitochondrial localizing p53 and interfered with the interaction with p53 in BcI-2 in cotransfection model (Fig. 5b). A recent study has shown that cytoplasmic and mitochondrial p53 binds members of the BcI-2 family in the response to CPT and directly triggers direct mitochondrial cell death[Mihara et al., Molecular Cell 11, 577-590 (2003)]. However, our present study indicates that Hades prevents the CPT-induced accumulation of p53 (Fig. 5e) and blocks the interaction between p53 and BcI-2 (Fig. 5f). Previous report has been identified that the interaction of p53 with BcI-2 protein and the subsequent loss of mitochondrial membrane potential are hallmarks of the transcription-independent cell death mechanism activated by p53[Tomita et al., J Biol Chem. 281, 8600-8606 (2006)]. Our results showed that Hades knockdown results in cytochrome C release from mitochondria upon CPT exposure (Fig. 5h) and loss of mitochondrial membrane potential in HCT116 p53^{+/+} cells, but not in HCT116 p53^{-/-} cells (Fig. 5j). FACS analysis also showed that the amount of apoptotic sub-G0 cell compartment is increased following CPT exposure in stable Hades knockdown MCF7 cells compared with control cells (Fig. 5i). Further, the present invention showed that Hades inhibits the exonuclear mechanism of p53 through ubiquitin-dependent proteasomal degradation to protect apoptosis induced by CPT. Based on the result, the present inventors propose that Hades negatively regulates the exonuclear mechanism of p53.

A hyper-ubiquitinated form of cytoplasmic p53 has reported in some cancers, such as neuroblastoma[Becker et al., Cell Death Duffer. 14, 1350-1360 (2007)]. This indicates that inhibition of exonuclear p53 function by ubiquitination may be related to tumorigenesis, since the exonuclear function is sufficient for p53 to manifest its tumor suppressor role under certain conditions[Talos et al., Cancer Res. 65, 9971-9981 (2005); Palacios et al., Cell Cycle 7, 2584-2590 (2008)]. We showed the elevated expression of Hades mRNA over 2 folds in liver biopsies from HCC patients (Fig 5k). This provides the first evidence suggesting that regulation of the exonuclear function of p53 may influence tumorigenesis. Although we do not currently define the precise mechanism that excessive Hades expression promotes general tumorigenesis by inhibiting the exonuclear function of p53, our findings suggest an alternative function of Hades for p53 tumor suppressor activity.

In conclusion, the present invention proposes that Hades is an E3 ligase for the p53 tumor suppressor and provide the first evidence to regulate p53 in the cytoplasm and modulates the exonuclear function of p53. Hades plays a unique role in the exonuclear p53 pathway, acting as a novel regulator in p53-dependent mitochondrial cell death pathway. Physiological significances of Hades need to be investigated.

### [Industrial Applicability]

As discussed above, the present inventors have found that the overexpressed Hades protein interacts with p53 to inhibit the exonuclear mechanism of p53 and the knowdown of Hades induces increase in the expression of p53, demonstrating that Hades is a negative regulator to p53. Therefore, it would be understood that the inhibition of Hades overexpressed in tumor cells contributes to tumor-supressive effects of p53. The drug candidates capable of modulating the expression of the Hades protein, inhibiting the actions of the Hades protein or inhibiting interecation between Hades and p53 are considered a promising anticancer drug.

## Claims

1. A composition for suppressing tumor comprising an expression or action inhibitor of Hades protein having an amino acid sequence of SEQ ID NO: 2 as an effective ingredient.

2. The composition for suppressing tumor according to claim 1, wherein the Hades is over-expressed in tumor cells.

3. The composition for suppressing tumor according to claim 1, wherein the expression or action inhibitor of Hades protein down regulates the transcription or translation of Hades gene or inhibits the action of Hades protein.

4. The composition for suppressing tumor according to claim 1, wherein the expression inhibitor of Hades protein is Hades siRNA (short interfering RNA) having a base sequence of SEQ ID NO: 4 or 5, or a gene transcribing the Hades siRNA.

5. The composition for suppressing tumor according to claim 1, wherein the action inhibitor of Hades protein is a substance inhibiting the interaction between Hades and p53.

6. The composition for suppressing tumor according to claim 5, wherein the interaction between Hades and p53 inhibits DNA binding of p53, transcription activity, stress-induced apoptosis and p53-induced apoptosis.

7. The composition for suppressing tumor according to claim 5, wherein the interaction betweem Hades and p53 induces ubiquitination of p53.

8. The composition for suppressing tumor according to claim 1, wherein the action inhibitor of Hades protein targets a RNIG-finger domain at the C-terminus of Hades.

9. A method for screening anti-cancer agent comprising:
(a) treating an agent in animal cells expressing Hades protein; and
(b) determining whether the expression of Hades protein decreases as compared to a control group in which the animal cells are not treated with the agent.

10. A method for screening anti-cancer agent comprising:
(a) contacting Hades protein comprising p53-binding site or its fragment and p53 in the existance of an agent; and
(b) determining whether the interaction between Hades and p53 is inhibited as compared to a control group in which the agent does not exist.

11. The Method for screening anti-cancer agent according to claim 10, wherein the fragment of Hades comprises p53-binding site (RING-finger domain at the C-terminus) having an amino acid sequence of SEQ ID NO: 3.

12. The Method for screening anti-cancer agent according to claim 10, wherein the interaction between Hades and p53 occures between p53-binding site (RING-finger domain at the C-terminus)of Hades and DNA-bindg domain of p53.

13. The Method for screening anti-cancer agent according to claim 10, wherein the interaction between Hades and p53 at the (b) step is detected by immunoprecipitation assay, GST pull-down assay and colony-formation assay.

14. An anti-cancer composition comprising the agent screened by the method for screening anti-cancer agent according to one of claims 9 to 13 as an effective ingredient.

15. A kit for screening anti-cancer agent which inhibits interaction between Hades and p53 comprising:
(a) a polypeptide comprising p53-binding site (RING-finger domain at the C-terminus)of Hades;
(a) a polypeptide comprising DNA-binding domain of p53; and
(c) an agent for detecting the interaction between the polypeptides.


<110>     Konkuk University Industrial Cooperation Corp

<120>     Use of Hades as a target for tumor suppressor

<160>     5

<170>     KopatentIn 2.0

<210>     1
<211>     1059
<212>     DNA
<213>     p53 binding gene

<400>     1
atggagagcg gagggcggcc ctcgctgtgc cagttcatcc tcctgggcac cacctctgtg          60

gtcaccgccg ccctgtactc cgtgtaccgg cagaaggccc gggtctccca agagctcaag         120

ggagctaaaa aagttcattt gggtgaagat ttaaagagta ttctttcaga agctccagga         180

aaatgcgtgc cttatgctgt tatagaagga gctgtgcggt ctgttaaaga aacgcttaac         240

agccagtttg tggaaaactg caagggggta attcagcggc tgacacttca ggagcacaag         300

atggtgtgga atcgaaccac ccacctttgg aatgattgct caaagatcat tcatcagagg         360

accaacacag tgccctttga cctggtgccc cacgaggatg gcgtggatgt ggctgtgcga         420

gtgctgaagc ccctggactc agtggatctg ggtctagaga ctgtgtatga gaagttccac         480

ccctcgattc agtccttcac cgatgtcatc ggccactaca tcagcggtga gcggcccaaa         540

ggcatccaag agaccgagga gatgctgaag gtgggggcca ccctcacagg ggttggcgaa         600

ctggtcctgg acaacaactc tgtccgcctg cagccgccca aacaaggcat gcagtactat         660

ctaagcagcc aggacttcga cagcctgctg cagaggcagg agtcgagcgt caggctctgg         720

aaggtgctgg cgctggtttt tggctttgcc acatgtgcca ccctcttctt cattctccgg         780

aagcagtatc tgcagcggca ggagcgcctg cgcctcaagc agatgcagga ggagttccag         840

gagcatgagg cccagctgct gagccgagcc aagcctgagg acagggagag tctgaagagc         900

gcctgtgtag tgtgtctgag cagcttcaag tcctgcgtct ttctggagtg tgggcacgtt         960

tgttcctgca ccgagtgcta ccgcgccttg ccagagccca agaagtgccc tatctgcaga        1020

caggcgatca cccgggtgat acccctgtac aacagctaa                               1059

<210>     2
<211>     352
<212>     PRT
<213>     p53 binding protein

<400>     2

Met Glu Ser Gly Gly Arg Pro Ser Leu Cys Gln Phe Ile Leu Leu Gly
1               5                   10                  15

Thr Thr Ser Val Val Thr Ala Ala Leu Tyr Ser Val Tyr Arg Gln Lys
            20                  25                  30

Ala Arg Val Ser Gln Glu Leu Lys Gly Ala Lys Lys Val His Leu Gly
        35                  40                  45

Glu Asp Leu Lys Ser Ile Leu Ser Glu Ala Pro Gly Lys Cys Val Pro
    50                  55                  60

Tyr Ala Val Ile Glu Gly Ala Val Arg Ser Val Lys Glu Thr Leu Asn
65                  70                  75                  80

Ser Gln Phe Val Glu Asn Cys Lys Gly Val Ile Gln Arg Leu Thr Leu
                85                  90                  95

Gln Glu His Lys Met Val Trp Asn Arg Thr Thr His Leu Trp Asn Asp
            100                 105                 110

Cys Ser Lys Ile Ile His Gln Arg Thr Asn Thr Val Pro Phe Asp Leu
        115                 120                 125

Val Pro His Glu Asp Gly Val Asp Val Ala Val Arg Val Leu Lys Pro
    130                 135                 140

Leu Asp Ser Val Asp Leu Gly Leu Glu Thr Val Tyr Glu Lys Phe His
145                 150                 155                 160

Pro Ser Ile Gln Ser Phe Thr Asp Val Ile Gly His Tyr Ile Ser Gly
                165                 170                 175

Glu Arg Pro Lys Gly Ile Gln Glu Thr Glu Glu Met Leu Lys Val Gly
            180                 185                 190

Ala Thr Leu Thr Gly Val Gly Glu Leu Val Leu Asp Asn Asn Ser Val
        195                 200                 205

Arg Leu Gln Pro Pro Lys Gln Gly Met Gln Tyr Tyr Leu Ser Ser Gln
    210                 215                 220

Asp Phe Asp Ser Leu Leu Gln Arg Gln Glu Ser Ser Val Arg Leu Trp
225                 230                 235                 240

Lys Val Leu Ala Leu Val Phe Gly Phe Ala Thr Cys Ala Thr Leu Phe
                245                 250                 255

Phe Ile Leu Arg Lys Gln Tyr Leu Gln Arg Gln Glu Arg Leu Arg Leu
            260                 265                 270

Lys Gln Met Gln Glu Glu Phe Gln Glu His Glu Ala Gln Leu Leu Ser
        275                 280                 285

Arg Ala Lys Pro Glu Asp Arg Glu Ser Leu Lys Ser Ala Cys Val Val
    290                 295                 300

Cys Leu Ser Ser Phe Lys Ser Cys Val Phe Leu Glu Cys Gly His Val
305                 310                 315                 320

Cys Ser Cys Thr Glu Cys Tyr Arg Ala Leu Pro Glu Pro Lys Lys Cys
                325                 330                 335

Pro Ile Cys Arg Gln Ala Ile Thr Arg Val Ile Pro Leu Tyr Asn Ser
            340                 345                 350

<210>   3
<211>   38
<212>   PRT
<213>   Hades C-terminal RING-finger domain

<400>   3
Cys Val Val Cys Leu Ser Ser Phe Lys Ser Cys Val Phe Leu Glu Cys
1               5                   10                  15

Gly His Val Cys Ser Cys Thr Glu Cys Tyr Arg Ala Leu Pro Glu Pro
            20                  25                  30

Lys Lys Cys Pro Ile Cys
        35

<210>   4
<211>   21
<212>   RNA
<213>   Hades siRNA anti-sense 1

<400>   4
gccucgagau aaaaauccct g                                                21

<210>   5
<211>   21
<212>   RNA
<213>   Hades siRNA anti-sense 2

<400>   5
uuuguccucu acacacacgt g                                                21